# EUROPEAN PATENT APPLICATION

(11) **EP 3 165 253 A1**
(43) Date of publication of application: **10.05.2017**
(21) Application number: 16197365.6
(22) Date of filing: 04.11.2016
(51) Int. Cl.: A61M 39/22, A61M 5/168, A61M 5/142, A61M 1/00, A61M 5/145

(54) **VALVE APPARATUS THAT REGULATES FLOW OF FLUID AND VACUUM PRESSURE**

(30) Priority: 06.11.2015 US 201562252001 P
(71) Applicant: Teleflex Medical Incorporated, Morrisville, North Carolina 27560 (US)
(72) Inventor: WHITLEY, Kenneth W., YOUNGSVILLE, NC North Carolina 27596 (US)
(74) Representative: Nguyen Van Yen, Christian

(57) **Abstract**

A valve apparatus includes a body including a first wall and a second wall, a chamber defined within the body, a valve member positioned in the chamber, a first conduit defined within the body to extend through the first wall and configured to be in fluid communication with the chamber and a source of fluid, a second conduit defined within the body to extend through the first wall and configured to be in fluid communication with the chamber and a source of vacuum pressure, and an actuator operatively connected to an end of the valve member extending through the body from the chamber. The actuator may position the valve member within the chamber to selectively form a first flow path with the first conduit that extends through the chamber and the second wall, and a second flow path with the second conduit that extends through chamber and the second wall.

## Description

### Technical Field

The present disclosure relates to fluid delivery systems, and, more particularly, to methods and systems for controlling a flow of fluid or an application of vacuum pressure through a fluid delivery device positioned within a patient, particularly with the aid of an ultrasound device.

### Background

Currently, ultrasound aided fluid injection procedures require two clinicians. A first clinician may hold a needle, which is attached by a conduit to a syringe, in one hand, and hold an ultrasound device, such as a probe, in a second hand. The first clinician may position the needle in a patient, and use the ultrasound device to visualize an area within the patient where medicine or an anesthetic is to be applied. While this procedure is being performed, the first clinician typically must periodically provide instructions to a second clinician to (1) pull a plunger of a syringe to "aspirate" the injection site, in order to check for accidental vessel puncture, and (2) depress the plunger to inject small boluses to help locate a needle. Thus, there is a need for a system which allows a single user to perform all of the functions necessary to inject a fluid into a patient, while currently being able to maneuver an ultrasound device to aid the injection procedure.

### Summary

The foregoing needs are met by the present disclosure, wherein according to certain aspects, a valve apparatus for regulating a flow of fluid and an application of vacuum pressure includes a first body including a first wall and a second wall, a chamber defined within the first body, a valve member positioned in the chamber, a first conduit defined within the body to extend through the first wall and configured to be in fluid communication with the chamber and one of a source of vacuum pressure and a source of fluid, a second conduit defined within the body to extend through the first wall and configured to be in fluid communication with the chamber and an other of the source of vacuum pressure and the source of fluid, and an actuator operatively connected to an end of the valve member extending through the first body from the chamber. The actuator may be configured to position the valve member within the chamber to selectively form a first flow path with the first conduit that extends through the chamber and the second wall of the first body, and a second flow path with the second conduit that extends through chamber and the second wall of the first body.

In accordance with other aspects of the present disclosure, the actuator may be configured to be operated by a hand of a user holding the valve apparatus and simultaneously holding the needle.

In accordance with yet other aspects of the present disclosure, a flexible strap may be attached to the first wall and the second wall of the first body, and configured to attach the valve apparatus to a hand of a user or an ultrasound device.

In accordance with other aspects of the present disclosure, the actuator may be configured to position the valve member within the chamber to form an obstruction between the second wall and at least one of the first conduit and the second conduit.

In accordance with yet other aspects of the present disclosure, the actuator may be a resilient actuator that may be biased from moving the valve member from a position corresponding to a formation of one of the first flow path, the second flow path, and the obstruction.

In accordance with yet other aspects of the present disclosure, a biasing member may be positioned within the first body of the valve apparatus.

In accordance with other aspects of the present disclosure, the biasing member may be configured to apply a biasing force to the valve member, and the valve member may be biased by the biasing force from moving from a position corresponding to a formation of one of the first flow path, the second flow path, and the obstruction.

In accordance with yet other aspects of the present disclosure, a third wall may extend from the first wall above the chamber, and a fourth wall may extend from the second wall above the chamber. In accordance with yet other aspects of the present disclosure, the actuator and the first end of the valve member may be positioned between the third wall and the fourth wall.

In accordance with yet other aspects of the present disclosure, a stopper may be movably positioned within a slot defined by the third wall or the fourth wall. In accordance with yet other aspects of the present disclosure, the valve member may be biased by the biasing force from moving from a first position corresponding to a formation of the second flow path, and may be moved against the biasing force into a second position corresponding to a formation of the obstruction. The stopper may be configured to move through the slot into a locked position above the actuator to maintain the actuator from moving under the biasing force from the second position.

In accordance with other aspects of the present disclosure, the biasing member may be positioned within the chamber.

In accordance with other aspects of the present disclosure, the biasing member may be positioned between the third wall and the fourth wall of the first body above the chamber.

In accordance with yet other aspects of the present disclosure, a third conduit may be defined by the second wall of the first body and configured to be in fluid communication with the chamber and a needle.

In accordance with yet other aspects of the present disclosure, a channel may be defined within a head of the valve member positioned within the chamber.

In accordance with other aspects of the present disclosure, a top surface of the head of the valve member may abut an internal surface of the first body that defines an upper wall of the chamber in a first position of the valve member. The channel may be blocked and the head of the valve member may form the obstruction in the first position.

In accordance with other aspects of the present disclosure, the valve member may be positioned within the chamber a first distance from the internal surface in a second position of the valve member, and the channel may be in fluid communication with the second conduit and the third conduit to form the second flow path through the head of the valve member in the second position.

In accordance with other aspects of the present disclosure, the valve member may be positioned within the chamber a second distance from the first internal surface greater than the first distance in a third position of the valve member, and the top surface of the head of the valve member and the internal surface of the first body may form a passage within the chamber that may be in fluid communication with the first conduit and the third conduit to form the first flow path.

In accordance with other aspects of the present disclosure, a fourth conduit may be defined within the body to extend through the second wall of the first body and configured to be in fluid communication with the chamber and the needle.

In accordance with other aspects of the present disclosure, a head of the valve member may be positioned with the chamber, and an outer surface of the head of the valve member may define a groove. The head of the valve member may form the obstruction between the first conduit and the third conduit in a first position of the valve member.

In accordance with yet other aspects of the present disclosure, a first biasing member may be positioned within the chamber and configured to apply a first biasing force on the head of the valve member. The valve member may be biased by the first biasing force from moving from the first position.

In accordance with other aspects of the present disclosure, a top surface of the head of the valve member may abut an internal surface of the first body that defines an upper wall of the chamber in the first position of the valve member.

In accordance with other aspects of the present disclosure, the head of the valve member may be positioned within the chamber a first distance from a first internal surface of the first body that defines an upper wall of the chamber in a second position of the valve member, and the groove may be in fluid communication with first conduit and the third conduit to form the first flow path.

In accordance with other aspects of the present disclosure, the valve member may be positioned within the chamber a second distance from the first internal surface greater than the first distance and the groove may be in fluid communication with the second conduit and the fourth conduit to form the second flow path in a third position of the valve member.

In accordance with other aspects of the present disclosure, the first biasing member may be attached to the first internal surface and a top surface of the head of the valve member. The biasing member may be held in tension in the second position of the valve member.

In accordance with yet other aspects of the present disclosure, the valve apparatus may include a protrusion extending from a second internal surface of the first body that defines a bottom of the chamber, a first stopper positioned within the chamber surrounding the first protrusion, and a second biasing member positioned within the chamber attached to the second internal surface and the first stopper. In accordance with other aspects of the present disclosure, the second biasing member may apply a second biasing force to the first stopper to bias the first stopper from moving from a position corresponding to a formation of an obstruction between the second conduit and fourth conduit.

In accordance with other aspects of the present disclosure, the first stopper may be in abutment with the head of the valve member in the second position of the valve member.

In accordance with other aspects of the present disclosure, the head of the valve member may define a recess extending from a bottom surface of the head of the valve member in a position corresponding to the first protrusion.

In accordance with other aspects of the present disclosure, the head of the valve member may be positioned within the chamber a second distance from the first internal surface greater than the first distance, the first stopper may be moved a distance towards the second internal surface, and the groove may be in fluid communication with the second conduit and the fourth conduit to form the second flow path in a third position of the valve member. In accordance with other aspects of the present disclosure, the bottom surface of the head of the valve member may be in abutment with the first stopper and the recess may receive the first protrusion in the third position.

In accordance with other aspects of the present disclosure, at least one second protrusion may extend from the bottom surface of the head of the valve member. The first stopper may be in abutment with the at least one second protrusion in the second position of the valve member.

In accordance with other aspects of the present disclosure, the head of the valve member may be positioned within the chamber a second distance from the first internal surface greater than the first distance in a third position of the valve member. In accordance with other aspects of the present disclosure, the first stopper may be moved a distance towards the second internal surface and the second conduit and the fourth conduit may be in fluid communication to form the second flow path through a passage defined by the bottom surface of the head of the valve member and the first stopper in the third position.

In accordance with other aspects of the present disclosure, the first protrusion may be in abutment with the bottom surface of the head of the valve member in the third position.

In accordance with yet other aspects of the present disclosure, the valve apparatus may include a second stopper positioned on the first protrusion below the first stopper. The first stopper may be in abutment with the second stopper in the third position.

In accordance with other aspects of the present disclosure, the actuator may include a handle that extends from a wall of the valve member that extends above the first conduit and the second conduit.

In accordance with other aspects of the present disclosure, the first wall of the first body may be perpendicular to the second wall.

In accordance with other aspects of the present disclosure, the actuator may be operated by a hand of a user holding the valve apparatus and simultaneously holding an ultrasound or the needle.

In accordance with yet other aspects of the present disclosure, the valve apparatus may include a second body attached to the second wall of the first body. The valve member may extend through the second wall into a recess defined within the second body.

In accordance with other aspects of the present disclosure, a channel may be defined within the valve member in fluid communication with a third conduit defined within the second body. In accordance with other aspects of the present disclosure, the valve member may be configured to rotate within the chamber of the first body and the recess of the second body.

In accordance with yet other aspects of the present disclosure, the valve apparatus may include a biasing member positioned with the second body and between the first body and the second body, and the biasing member may maintain the valve member in a first position. In accordance with yet other aspects of the present disclosure, the channel may be positioned between the first conduit and the second conduit along a rotational direction of movement of the valve member in the first position.

In accordance with other aspects of the present disclosure, the actuator may be configured to rotate the valve member in a first direction a first distance from the first position to a second position, and the channel may be in fluid communication with the first conduit in the second position.

In accordance with other aspects of the present disclosure, the biasing member may be formed from an elastic material. In accordance with other aspects of the present disclosure, the biasing member may be configured to apply a restoring force in a second direction opposite to the first direction to the valve member in the second position. The biasing member may be configured to move the valve member from the first position to the second position with the restoring force.

In accordance with other aspects of the present disclosure, a fluid injection system includes a fluid source, a vacuum source, a needle, an ultrasound device, and a valve apparatus. The valve apparatus includes a body including a first wall and a second wall, a chamber defined within the body, a valve member positioned in the chamber, a first conduit defined within the body to extend through first wall in fluid communication with the chamber and one of the fluid source and the vacuum source, a second conduit defined within the body to extend through the first wall in fluid communication with the chamber and an other of the fluid source and the vacuum source, and an actuator operatively connected to an end of the valve member extending through the first body from the chamber. In accordance with other aspects of the present disclosure, the actuator may be configured to position the valve member within the chamber to selectively form a first flow path with the first conduit that extends through the chamber in fluid communication with the needle, and a second flow path with the second conduit that extends through chamber in fluid communication with the needle. In accordance with other aspects of the present disclosure, the actuator may be configured to be operated by a hand of a user holding the valve apparatus and simultaneously holding the ultrasound or the needle.

In accordance with other aspects of the present disclosure, a method of controlling a flow of fluid and an application of vacuum pressure through a needle with a valve apparatus includes removably mounting the valve apparatus to a first hand of a user, connecting a pressurized source of the fluid to a first conduit of the valve apparatus that may be defined within a body of the valve apparatus that further defines a chamber, connecting a source of vacuum pressure to a second conduit of the valve apparatus that may be defined within the body, connecting the needle to a third conduit of the valve apparatus that may be defined within the body, holding the needle with at least a first finger and a second finger of the first hand, operating an actuator operatively connected to a valve member positioned in the chamber with at least a third finger of the first hand to position the valve member within the chamber to selectively form a first flow path that includes the first conduit, the chamber, and the third conduit for supplying the flow of fluid to the needle, and a second flow path that includes the second conduit, the chamber, and the third conduit for applying the vacuum pressure through the needle.

In accordance with yet other aspects of the present disclosure, the method includes positioning the needle relative to an injection site of a patient, operating the actuator with the at least third finger to position the valve member within the chamber to a first position corresponding to a formation of the second flow path to apply the vacuum pressure to the needle and aspirate the injection site within the patient, and operating the actuator with the at least third finger to move the valve member within the chamber from the first position corresponding to the formation of the second flow path to a second position corresponding to a formation of the first flow path to supply the flow of fluid to the injection site.

In accordance with yet other aspects of the present disclosure, the method includes holding an ultrasound device in a second hand of the user, injecting the needle into a patient with the first hand, guiding the ultrasound device along the skin of the patient with the second hand, and guiding the needle within the patient to an injection site within the patient according to a view generated by the ultrasound device.

In accordance with yet other aspects of the present disclosure, the method includes operating the actuator with the at least third finger to position the valve member within the chamber to a first position corresponding to a formation of the second flow path to apply the vacuum pressure to the injection site, and operating the actuator with the at least third finger to move the valve member within the chamber from the first position corresponding to the formation of the second flow path to a position corresponding to a formation of the first flow path to supply the flow of fluid to the injection site.

In accordance with yet other aspects of the present disclosure, the method includes operating the actuator with the at least third finger to position the valve member within the chamber to a first position corresponding to a formation of the first flow path to supply the flow of fluid to the injection site, and operating the actuator with the at least third finger to position the valve member within chamber from the second position corresponding to the formation of the first flow path to a second position corresponding to a formation of the second flow path to apply the vacuum pressure to the injection site.

In accordance with other aspects of the present disclosure, operating the actuator with the at least third finger includes applying a downward force to the actuator and withdrawing the downward force.

In accordance with other aspects of the present disclosure, positioning the valve member in the first position includes moving the valve member into abutment with a stopper positioned within the chamber, and positioning the valve member in the second position includes moving the valve member and the stopper within the chamber.

In accordance with yet other aspects of the present disclosure, the method includes operating the actuator with the at least third finger to position the valve member within the chamber to a position corresponding to a formation of an obstruction between the third conduit and both of the first conduit and the second conduit.

In accordance with other aspects of the present disclosure, operating the actuator with the at least third finger to position the valve member in the first position includes rotating the actuator in a first direction, and operating the actuator with the at least third finger to position the valve member in the second position includes rotating the actuator in a second direction opposite to the first direction.

In accordance with other aspects of the present disclosure, a method of controlling a flow of fluid and an application of vacuum pressure to a needle with a valve apparatus includes removably mounting the valve apparatus to an ultrasound device, connecting a pressurized source of the fluid to a first conduit of the valve apparatus that may be defined within a body of the valve apparatus that may further define a chamber, connecting a source of vacuum pressure to a second conduit of the valve apparatus that may be defined within the body, connecting the needle to a third conduit of the valve apparatus that may be defined within the body, holding a needle with a first hand of a user, holding a combination of the valve apparatus and the ultrasound device in a second hand of a user, and operating an actuator operatively connected to a valve member of the valve apparatus positioned within the chamber with at least a first finger of the second hand to position the valve member to selectively form a first flow path that includes the first conduit, the chamber, and the third conduit for supplying the flow of fluid to the needle, and a second flow path that includes the second conduit, the chamber, and the third conduit for applying the vacuum pressure to the needle.

In accordance with yet other aspects of the present disclosure, the method includes injecting the needle into a patient with the first hand, guiding the ultrasound device along the skin of the patient with the second hand, and guiding the needle within the patient to an injection site within the patient according to a view generated by the ultrasound device.

There has thus been outlined, rather broadly, certain aspects of the present disclosure in order that the detailed description herein may be better understood, and in order that the present contribution to the art may be better appreciated.

In this respect, before explaining at least one embodiment of the invention in detail, it is to be understood that the invention is not limited in its application to the details of the construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### Brief Description of the Drawings

FIG. 1 illustrates a system for regulating a supply of fluid and an application of vacuum pressure through a needle, according to an aspect of the present disclosure.
FIG. 2 illustrates a valve apparatus mounted on to a hand, according to an aspect of the present disclosure.
FIG. 3 illustrates a valve apparatus mounted on to an ultrasound device, according to an aspect of the disclosure.
FIGS. 4A-C illustrate a front cross sectional view taken along section line 4-4, of the valve apparatus of FIG. 1 positioned in neutral and actuated positions.
FIGS. 5A and B illustrate a front cross sectional view taken along section line 5-5, of the valve apparatus of FIG. 1.
FIGS. 6A-C illustrate a front cross sectional view of a valve apparatus positioned in neutral and actuated positions, according to an aspect of the present disclosure.
FIGS. 7A-C illustrate a front cross sectional view of a valve apparatus positioned in neutral and actuated positions, according to an aspect of the present disclosure.
FIGS. 8A-C illustrate a front cross sectional view of a valve apparatus positioned in neutral and actuated positions, according to an aspect of the present disclosure.
FIGS. 9A-C illustrate a front cross sectional view of a valve apparatus positioned in neutral and actuated positions, according to an aspect of the present disclosure.
FIGS. 10A-C illustrate a front cross sectional view of a valve apparatus positioned in neutral and actuated positions, according to an aspect of the present disclosure.
FIG. 11 illustrates a front cross sectional view of a valve apparatus, according to an aspect of the present disclosure.
FIG. 12 illustrates a cross sectional view of a valve apparatus, according to an aspect of the present disclosure.
FIG. 13 illustrates a top cross sectional view of the valve apparatus of Fig. 12, taken along section line 13-13.
FIG. 14 illustrates a top cross sectional view of the valve apparatus of Fig. 12, taken along section line 14-14.
FIG. 15 illustrates a top cross sectional view of the valve apparatus of Fig. 12, taken along section line 15-15.
FIG. 16 illustrates a cross sectional view of a valve apparatus, according to an aspect of the present disclosure.
FIGS. 17A and 17B illustrate a top cross sectional view taken along section line 17-17, of the valve apparatus of FIG. 16 in actuated positions.
FIGS. 18A and 18B illustrate a top cross sectional view taken along section line 18-18, of the valve apparatus of FIG. 17 in actuated positions.
FIG. 19 illustrates a perspective view of a valve apparatus, according to an aspect of the present disclosure.
FIG. 20 illustrates an exploded view of the valve apparatus of FIG. 19.
FIG. 21 illustrates a bottom perspective view of the valve apparatus of FIG. 19.
FIGS. 22A and 22B illustrate a top view of the valve apparatus of FIG. 19.
FIGS. 23A-C illustrate a cross-sectional view of the valve apparatus of FIG. 19 in neutral and actuated positions.

### Detailed Description

Aspects of the disclosure will now be described in detail with reference to the drawings, wherein like reference numerals designate identical or corresponding parts throughout the several views, unless specified otherwise.

It is noted that as used in the specification and the appending claims the singular forms "a," "an," and "the" can include plural references unless the context clearly dictates otherwise.

Unless specified otherwise, the terms "substantial" or "substantially" as used herein mean "considerable in extent," or "largely but not necessarily wholly that which is specified."

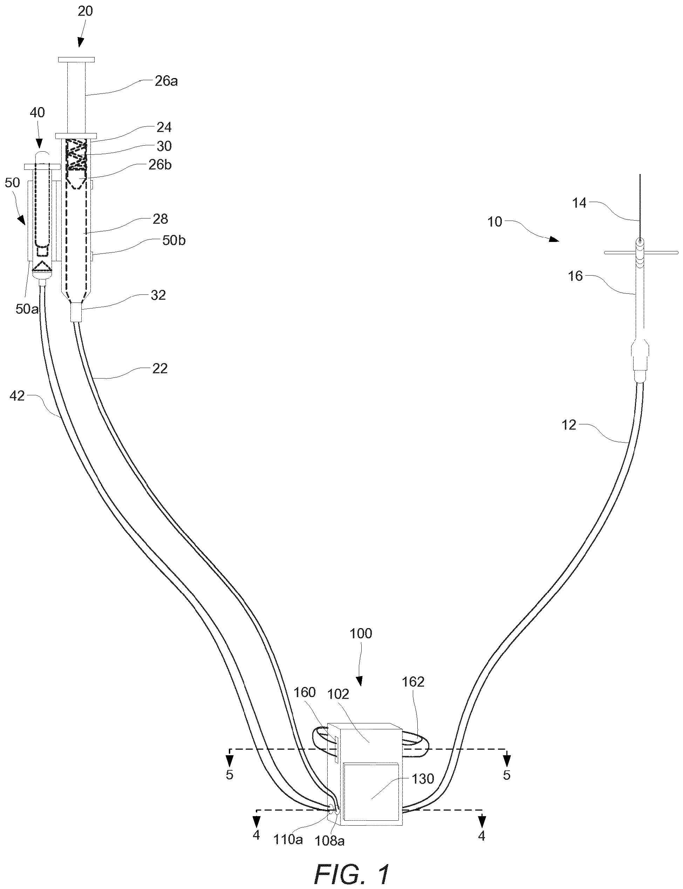

FIG. 1 illustrates a system for regulating a supply of fluid and an application of vacuum pressure to a needle device 10, according to an aspect of the present disclosure. The needle device 10 receives a supply of fluid from a syringe 20, and a vacuum pressure generated by a vacuum device 40 is applied through the needle device 10, through a valve apparatus 100. The needle device 10 includes a needle 14 in fluid communication with a delivery conduit 12 which is attached to a cylindrical body 16 of the needle device 10. One of ordinary skill in the art will recognize the delivery conduit 12 may be a tube or other fluid carrying structure suitable for the conveyance of a fluid, such as a medication or type of anesthetic, into a patient receiving medical aid or treatment.

The syringe 20 is in fluid communication with a first conduit 22 and includes a housing 24 through which a plunger actuator 26a extends. A plunger 26b is attached to an end of the plunger actuator 26a and positioned within a fluid supply chamber 28 defined within the housing 24. The plunger 26b is normally biased within the fluid supply chamber 28 by a syringe biasing member 30 to move towards an outlet end 28a. The syringe biasing member 30 may be a spring or other device that elastically applies a force to the plunger 26b. When the plunger actuator 26a, and thus the plunger 26b, is withdrawn to a retracted position, for example during a fluid filling operation, the syringe biasing member 30 compresses. The compressed syringe biasing member 30 exerts a force on the plunger 26b that may move the plunger 26b down in the fluid supply chamber 28 from the retracted position and create a constant source of pressure.

Accordingly, the combination of the plunger 26b and the syringe biasing member 30 cause the fluid supply chamber 28 to be pressurized. As a result, fluid within the fluid supply chamber 28 is normally forced by the plunger 26b through the fluid supply chamber 28. A self-sealing valve 32 (e.g. a luer-type valve) connected to the syringe 20, will remain closed and prevent fluid from flowing under pressure from the fluid supply chamber 28 until it is attached to a mating luer (not shown) on a proximal end of the first conduit 22.

The vacuum device 40 is in fluid communication with a second conduit 42 and attached to the syringe 20 by a clip device 50. The clip device 50 includes a holder 52 that receives, and secures to, the vacuum device 40. The clip device 50 includes arms 54 which extend from a side of the holder 52 and may lock or elastically deform around the housing 24 to secure the syringe 20 to the vacuum device 40. According to one aspect of the present disclosure, the vacuum device 40 may be a vacutainer or a reversed-spring syringe. Alternatively, a source of vacuum pressure may be provided through a vacuum supply line, for example, in a hospital.

As illustrated in FIG. 1, the first conduit 22 is connected to a first external port 108a in a wall of a body 102 of the valve apparatus 100. The second conduit 42 is connected to a second external port 110a in the wall of the body 102 of the valve apparatus 100. However, the second external port 110a may also be left open to atmosphere. The delivery conduit 12 is connected to a port in another wall of the body 102 not shown. The valve apparatus 100 includes an actuator 130 that may be selectively operated to control a fluid communication between the delivery conduit 12 and the first conduit 22 or the second conduit 42.

A slot 160 extends through the body 102 of the valve apparatus 100 and accommodates a strap 162. One of ordinary skill in the art will recognize the slot 160 may be of any cross-section (e.g. circular, square, rectangular, etc.), and the strap 162 may be of any type capable of being run through the slot 160 and used to mount the valve apparatus 100 on another element, such as a hand or ultrasound device as described in more detail below.

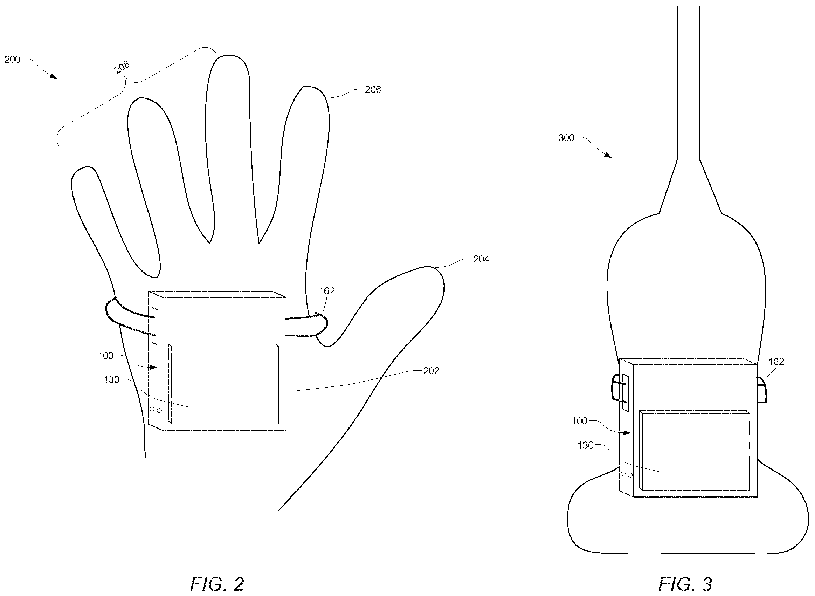

FIG. 2 illustrates the valve apparatus 100 mounted on to a hand 200, according to an aspect of the present disclosure. The valve apparatus 100 is sized such that valve apparatus 100 may fit into a palm 202 of a user. In particular, the strap 162 may be expanded or separated and reattached to wrap around the hand 200 such that no further action is necessary for the valve apparatus 100 to remain on the hand 200. In addition, the actuator 130 is sized and arranged such that any one of a set of fingers 208 not including a thumb 204 or an index finger 206, may be used to operate the valve apparatus 100 by applying a downward force on the actuator 130 and selectively placing the delivery conduit 12 in fluid communication with the first conduit 22 or the second conduit 42.

One of ordinary skill in the art will recognize that allowing a user to operate the actuator 130 with one, two, or even all three of the set of fingers 208, allows both the thumb 204 and the index finger 206 to be free to be used to hold and/or operate another device. For example, the thumb 204 and the index finger 206 could be used to hold the needle device 10 of FIG. 1. It will be also be appreciated that if desired, the user may utilize either the thumb 204 or the index finger 206 to apply a force on the actuator 130 to operate the valve apparatus 100.

FIG. 3 illustrates the valve apparatus 100 mounted on to an ultrasound device 300, according to an aspect of the present disclosure. The valve apparatus 100 is sized to have a width such that the valve apparatus 100 may be attached to a surface of the ultrasound device 300 and not extend beyond the width of the ultrasound device 300 as illustrated in FIG. 3. Further, the strap 162 may be expanded or separated and reattached to wrap around the ultrasound device 300 such that no further action is necessary for the valve apparatus 100 to remain thereon. The valve apparatus 100 may be positioned on the ultrasound device so that a user can support the ultrasound device with a thumb of one hand and operate the valve apparatus 100 with the remaining fingers on the same hand, our vice versa. It will be appreciated that the ultrasound device 300 is only an example of a device to which the valve apparatus 100 may be attached using the strap 162.

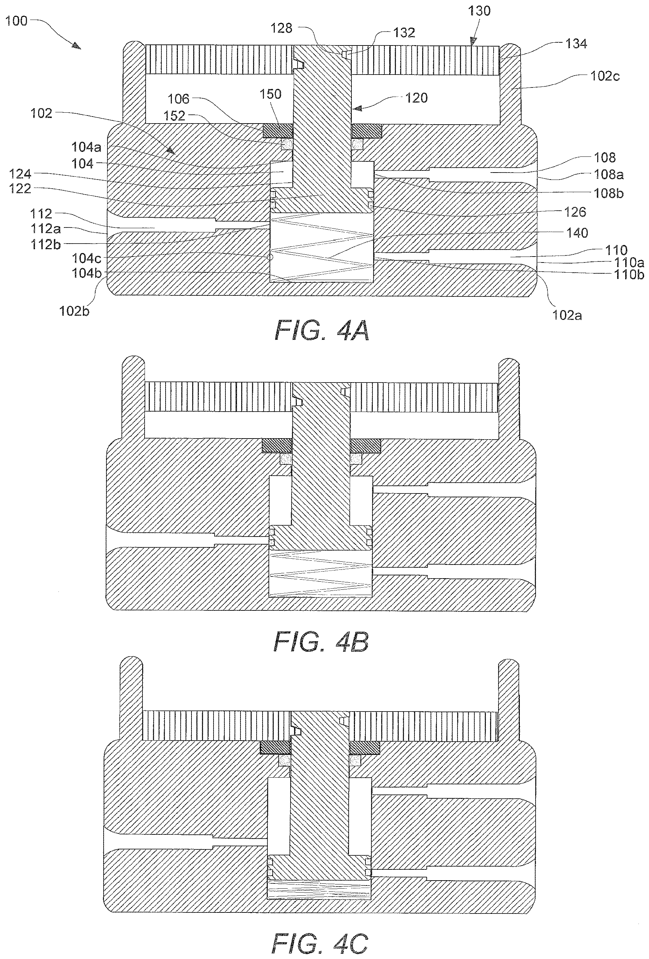

A user may implement the system of FIG. 1 by attaching the valve apparatus 100 in the manner illustrated in FIG. 2 or FIG. 3, in order to perform a procedure such as an injecting a peripheral nerve block medication into a patient aided by the use of an ultrasound device such as the ultrasound device 300 illustrated in FIG. 3. As will be explained in more detail with reference to FIG. 4, and the various embodiments of a valve apparatus described herein, each of which being capable of being incorporated in the system of FIG. 1, the valve apparatus 100 may allow a single user, such as a clinician or other medical professional, to use a single hand to perform all of the functions necessary to inject a fluid into a patient, while having another hand completely free to operate an ultrasound device, such as an ultrasound probe.

More specifically, with each embodiment of a valve apparatus described herein, one user will be able to (1) with a first hand, manipulate the ultrasound device 300, and thereby obtain a visual representation of where the needle 14 of the needle device 10 is located relative to a desired location within a patient; (2) with a second hand, maneuver the needle 14 within the patient to and around the desired location per the view the user is able to independently obtain via the user's operation of the ultrasound device; and (3) with either of the first hand or the second hand, depending on whether the valve apparatus 100 is attached to ultrasound device 300 held in the first hand, or attached to the second hand manipulating the needle device 10, control a flow of medication or anesthetic to the needle 14 from the syringe 20 using the actuator 130.

An additional advantage of the valve apparatus 100 is provided by the second external port 110a, particularly as utilized in the system of FIG. 1. With the same hand which operates the valve apparatus 100, or any other embodiment of a valve apparatus described herein, to supply fluid to the patient, a vacuum pressure may be applied through the needle 14. The user may aspirate an injection site without having to attempt to retract the plunger actuator 26a, or request assistance from another person. In practice, the user will be able to intermittently operate the actuator 130 to aspirate an injection cite and check for blood or cerebral spinal fluid (CSF). Accordingly, the user will be able to determine if the needle 14 has punctured a blood vessel or a nerve, and needs to be withdrawn and relocated, without assistance from an additional person.

FIGS. 4A-3C illustrate a front cross sectional view taken along section line 4-4, of the valve apparatus 100 of FIG. 1 positioned in neutral and actuated positions. With reference to FIG. 4A, the valve apparatus 100 includes the body 102 which defines a chamber 104, a recess 106, a first inlet conduit 108, a second inlet conduit 110, and an outlet conduit 112. The chamber 104 is defined by an upper wall 104a, a bottom wall 104b, and side walls 104c formed within the body 102. The first inlet conduit 108 extends through the body 102 and includes the first external port 108a in a first wall 102a, and a first internal port 108b in one side wall 104c of the chamber 104. The second inlet conduit 110 extends through the body 102 and includes the second external port 110a in the first wall 102a, and a second internal port 110b in the one side wall 104c of the chamber 104. The outlet conduit 112 extends through the body 102 from a third external port 112a in a second wall 102b of the body 102, to a third internal port 112b in another side wall 104c of the chamber 104.

The valve apparatus 100 includes a valve member 120 operatively attached to the actuator 130. An end of the valve member 120 includes a valve head 122 positioned within the chamber 104. A rod 124 extends from the valve head 122 through the chamber 104 and the body 102 of the valve apparatus 100. Sealing members 126 (e.g. O-rings) are positioned within grooves formed within the valve head 122 and maintain a seal between the valve head 122 and the side walls 104c of the chamber 104. An end of the rod 124 opposite to an end attached to the valve head 122 includes recesses 128. The recesses 128 receive protrusions 132 of the actuator 130 to provide an interlocking attachment between the rod 124 and the actuator 130.

The actuator 130 is positioned between extension walls 102c of the body 102. The extension walls 102c extend vertically from the first wall 102a and the second wall 102b on opposite sides of the chamber 104. The actuator 130 includes side walls 134 that slide along inner surfaces of the extension walls 102c to keep a combination of the actuator 130 and valve member 120 aligned for upward and downward movement.

The valve member 120 is biased to move in an upward direction by a biasing member (e.g. a spring) positioned within the chamber 104. While the biasing member 140 presses against the valve head 122, the rod 124 is surrounded by a bushing 150 and a sealing member 152 (e.g. an O-ring) above the chamber 104 and extends through the recess 106 formed in the body 102. The bushing 150 is received in the recess 106 to hold the rod 124 in alignment and guide an upward and downward movement of the valve member 120 resulting from a biasing force exerted on the valve head 122 by the biasing member 140, or an external downward force applied to the actuator 130 by, for example, a user such as a clinician.

FIG. 4A illustrates the valve apparatus 100 in a neutral position in which a flow path is formed through the chamber 104 with the second inlet conduit 110 and the outlet conduit 112. Therefore, in a natural ("relaxed") state corresponding to the neutral position of the valve apparatus 100, the flow path is formed by the second inlet conduit 110, the chamber 104, and the outlet conduit 112. As a result, a vacuum pressure will normally be applied through the needle 14 from the vacuum device 40 where the vacuum device 40 is fluidly connected through the second conduit 42 to the second external port 110a, and the second external port 110a is in fluid communication with the delivery conduit 12 through the second inlet conduit 110, the outlet conduit 112, and the chamber 104.

FIG. 4B illustrates the valve apparatus in 100 in a first actuated position after an external force has been applied to the actuator 130 sufficient to move the valve member 120 within the chamber 104 so that the valve head 122 obstructs the third internal port 112b. The external force will be transmitted from the actuator 130 through the valve member 120 and exerted by the valve head 122 on the biasing member 140 which may compress so that the valve head 122 will move downward within the chamber 104. A user may hold the actuator 130 in a position corresponding to a formation of an obstruction of the third internal port 112b so that a fluid from the syringe 20 will not be supplied, and the vacuum pressure from the vacuum device 40 will not be applied through the needle 14.

FIG. 4C illustrates the valve apparatus in 100 in a second actuated position after an external force has been applied to the actuator 130 sufficient to move the valve member 120 within the chamber 104 so that the valve head 122 obstructs the second internal port 110b. The external force will be transmitted from the actuator 130 through the valve member 120 and exerted by the valve head 122 on the biasing member 140 which will be compressed a substantially maximum amount. The first internal port 108b and the third internal port 112b will not be obstructed by the valve head 122, and therefore be in fluid communication through the chamber 104 to form a flow path. A pressurized supply of fluid provided from the syringe 20 with the operation of the syringe biasing member 30 acting on the plunger 26b, will flow through the first conduit 22 into to the first inlet conduit 108 and through the chamber 104. From the chamber 104, the fluid will flow under pressure through the outlet conduit 112 and the delivery conduit 12 to the needle 14.

It will be appreciated that the first conduit 22 may be connected to the second external port 110a, and the second conduit 42 may be connected to the first external port 108a. In such a configuration, fluid will normally be supplied from the syringe 20 through the valve apparatus 100 positioned in the neutral position illustrated in FIG. 4A.

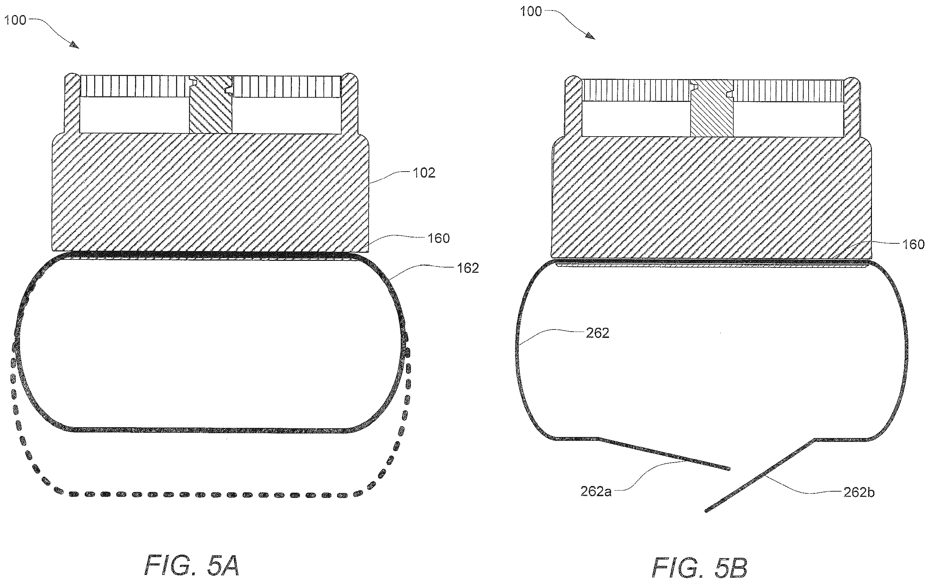

FIGS. 5A and 5B illustrate a front cross sectional view taken along section line 5-5, of the valve apparatus of FIG. 1. FIGS. 5A and 5B illustrate two types of straps (162, 262) that may be incorporated with the valve apparatus. Each strap (162, 262) is received in the slot 160 and extends through the body 102. In FIG. 5A, the strap 162 is an elastic band which may expand according to the size of a hand or device the valve apparatus 100 is mounted to. The valve apparatus 100 may be provided with an opening mechanism (not shown) which allows the strap 162 to be installed and removed from the slot 160. Alternatively, the body 102 may be formed through a manufacturing process around the strap 162. In FIG. 5B, the strap 262 is detachable, such that a first strip 262a can be detached from a second strap 262b. In one exemplary embodiment, the strap 262 may include Velcro strips which can be easily detached and reattached. One of ordinary skill in the art will appreciate other types of detachable straps may be inserted through the slot 160 according to the present disclosure.

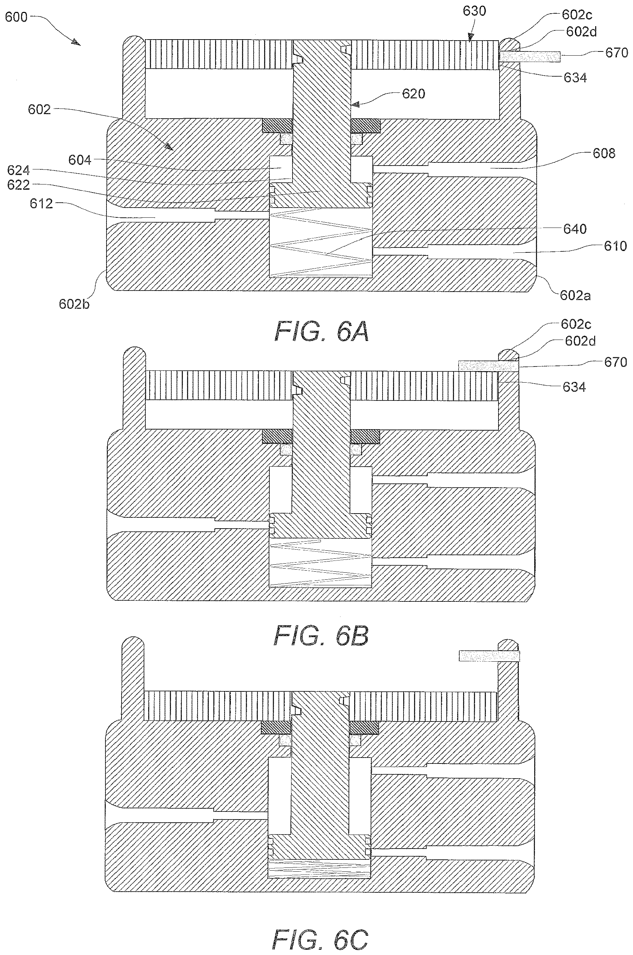

FIGS. 6A-C illustrate a front cross sectional view of a valve apparatus 600 positioned in neutral and actuated positions, according to an aspect of the present disclosure. With reference to FIG. 6A, the valve apparatus 600 includes a body 602 which defines a chamber 604, a first inlet conduit 608, a second inlet conduit 610, and an outlet conduit 612. The first inlet conduit 608 and the second inlet conduit 610 extend through the body 602 to a first wall 602a, and the outlet conduit 612 extends through the body 602 to a second wall 602b. A valve member 620 is operatively attached to an actuator 630, and includes a valve head 622 positioned within the chamber 604. The valve member 620 further includes a rod 624 that extends from the valve head 622 through the chamber 604 and the body 602 of the valve apparatus 600.

The valve apparatus 600 operates in the same manner of the valve apparatus 100 illustrate in FIGS. 4A-C. Like the valve apparatus 100 of FIGS. 4A-C, the actuator 630 of the valve apparatus 600 is positioned between extension walls 602c of the body 602 which extend from the first wall 602a and the second wall 602b on opposite sides of the chamber 604. The actuator 630 includes side walls 634 that slide along inner surfaces of the extension walls 602c to keep a combination of the actuator 630 and valve member 620 aligned for upward and downward movement. In addition, at least one extension wall 602c includes a slot 602d defined therein, which receives an external stopper 670.

The external stopper 670 is located in a first external stopper position in FIG. 6A with one end facing one side wall 634 of the actuator 630. Similar to the inner surfaces of the extension walls 602c, the side wall 634 of the actuator 630 can slide along the end of the external stopper 670 in the first external stopper position. During operation, the actuator 630 may move downward under the application of a downward force and the side wall 634 of the actuator 630 will no longer be positioned adjacent to the slot 602d. Accordingly, as illustrated in FIG. 6B, the external stopper 670 may be moved through the slot 602d into a second external stopper position.

In the second external stopper position, the external stopper 670 extends into a space between the inner surfaces of the extension walls 602c above the actuator 630. As illustrated in FIG. 6B, the valve head 622 obstructs fluid communication between the outlet conduit 612 and both of the first inlet conduit 608 and the second inlet conduit 610. In addition, with the external stopper located 670 in the second position, a biasing member 640 positioned within the chamber 604 exerts an upward biasing force on the valve head 622 which is transmitted to the actuator 630. Once a downward force being applied by a user is relieved, the biasing force of the biasing member 640 causes the actuator 630 to remain in abutment with a bottom surface of the external stopper 670 located in the second external stopper position. Thus the actuator 630 and the valve member 620 may remain locked in a first actuated position illustrated in FIG. 6B, blocking fluid communication between the outlet conduit 612 and both of the first inlet conduit 608 and the second inlet conduit 610.

A user may move the external stopper 670 once the actuator 630 is moved a sufficient distance. Alternatively, the external stopper 670 may be spring loaded so that once the slot 602d is no longer blocked by the side wall 634, the external stopper 670 automatically moves through the slot 602d under the force of a biasing mechanism (not shown). Thus a user, such as clinician, can focus on positioning the needle 14 within a patient with the aid of the ultrasound device 300, without fluid flowing or the vacuum pressure being applied through the needle 14. Once the actuator 630 is in the locked position, the user no longer has to apply a force to the actuator to maintain an obstructing position of the valve head 622.

When a fluid supply is required from the syringe 20, a down force applied to the actuator 630 in the locked position will move the actuator downward into a second actuated position illustrated in FIG. 6C. As a result, the first inlet conduit 608 will be in fluid communication through the chamber 604 with the outlet conduit 612. When a vacuum pressure is to be applied through the needle 14, the external stopper 670 can be moved into the first external stopper position. This will allow the actuator 630 to be moved to a neutral position illustrated in FIG. 6A by the force applied to the valve head 622 by the biasing member 640.

One of ordinary skill will appreciate that the slot 602d may be positioned anywhere along a vertical axis within at least one of the extension walls 602c. Thus, the slot 602d and the external stopper 670 can be located closer to the first wall 602a and/or the second wall 602b so that the actuator 630 and the valve member 620 can be locked in a second actuated position illustrated in FIG. 6C.

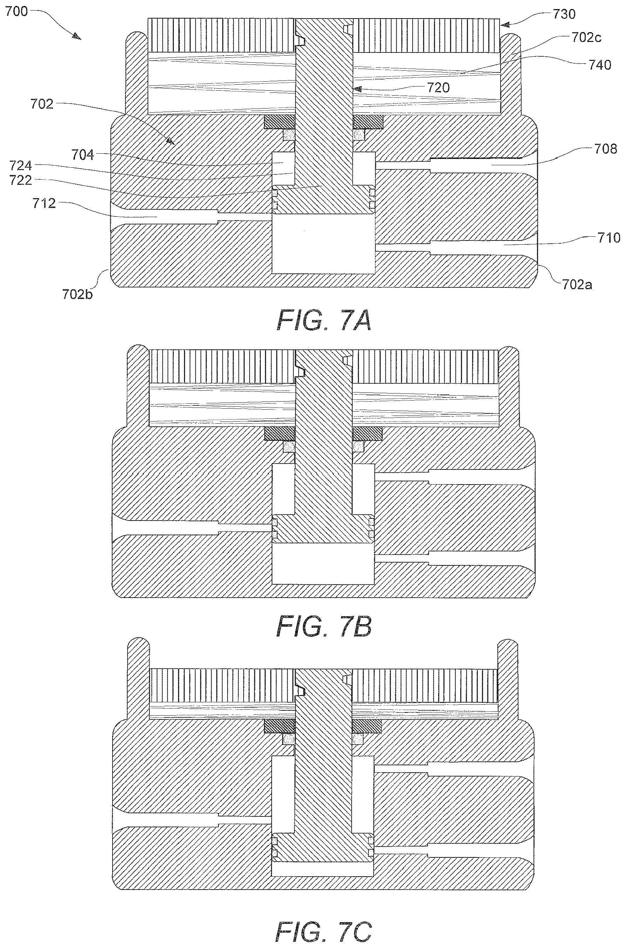

FIGS. 7A-C illustrate a front cross sectional view of a valve apparatus 700 positioned in neutral and actuated positions, according to an aspect of the present disclosure. With reference to FIG. 7A, the valve apparatus 700 includes a body 702 which defines a chamber 704, a first inlet conduit 708, a second inlet conduit 710, and an outlet conduit 712. The first inlet conduit 708 and the second inlet conduit 710 extend through the body 702 to a first wall 702a, and the outlet conduit 712 extends through the body 702 to a second wall 702b. A valve member 720 is operatively attached to an actuator 730, and includes a valve head 722 positioned within the chamber 704. The valve member 720 further includes a rod 724 that extends from the valve head 722 through the chamber 704 and the body 702 of the valve apparatus 700.

Unlike the previous valve apparatuses (100, 600), the valve apparatus 700 of FIGS. 7A-C includes a biasing member 740 located outside of the chamber 704. The biasing member 740 surrounds the rod 724 and is positioned in a space between wall extensions 702c that extend from the first wall 702a and the second wall 702b. The biasing member 740 exerts an upward force on the actuator 730 such that in a neutral position illustrated in FIG. 7A, a fluid passage is defined by the chamber 704 between the second inlet conduit 710 and the outlet conduit 712.

FIG. 7B illustrates the valve apparatus 700 in a first actuated position with an external force being applied to the actuator 730 against a biasing force of the biasing member 740. The actuator 720 is depressed a sufficient distance so the valve head 722 obstructs fluid communication between the outlet conduit 712 and both of the first inlet conduit 708 and the second inlet conduit 710. A user may hold the actuator 730 in a position corresponding to a formation of the obstruction so that a fluid from the syringe 20 will be not supplied and the vacuum pressure from the vacuum device 40 will not be applied through the needle 14.

FIG. 7C illustrates the valve apparatus in 700 in a second actuated position after an external force has been applied to the actuator 730 sufficient to move the valve member 720 within the chamber 104 so that the valve head 722 obstructs the second inlet conduit 710. The external force will be transmitted from the actuator 730, against biasing member 740 outside of the body 702. The first inlet conduit 708 is in fluid communication with the outlet conduit 712 through the chamber 704.

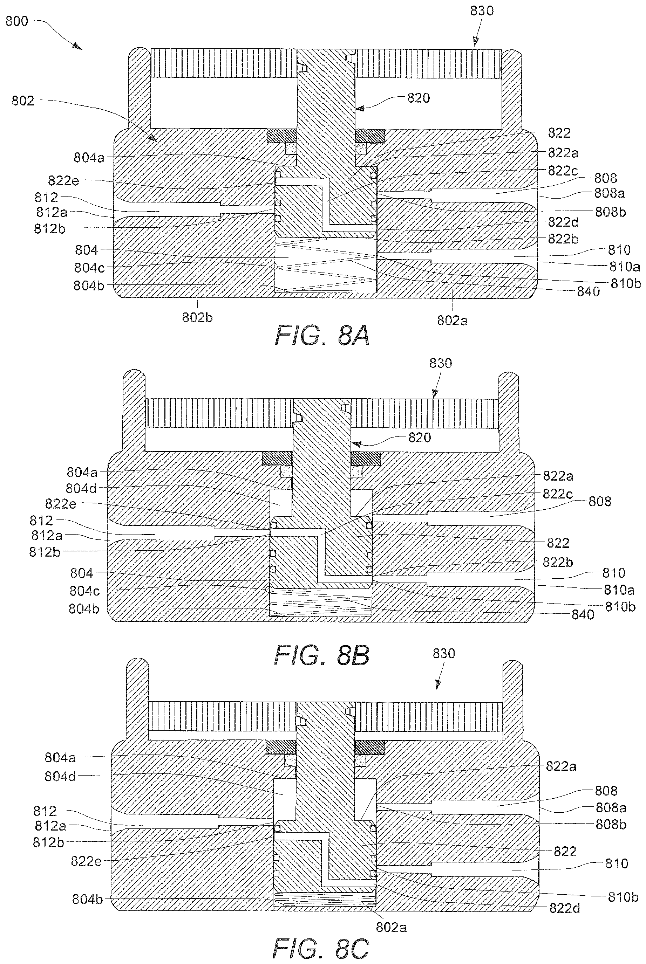

FIGS. 8A-C illustrate a front cross sectional view of a valve apparatus 800 positioned in neutral and actuated positions, according to an aspect of the present disclosure. With reference to FIG. 8A, the valve apparatus 800 includes a body 802 which defines a chamber 804, a first inlet conduit 808, a second inlet conduit 810, and an outlet conduit 812. The chamber 804 is defined by an upper wall 804a, a bottom wall 804b, and side walls 804c formed within the body 802. The first inlet conduit 808 extends through the body 802 and includes a first external port 808a in a first wall 802a, and a first internal port 808b in one side wall 804c of the chamber 804. The second inlet conduit 810 extends through the body 802 and includes a second external port 810a in the first wall 802a, and a second internal port 810b in the one side wall 804c of the chamber 804. The outlet conduit 812 extends through the body 802 from a third external port 812a in a second wall 802b of the body 802 to a third internal port 812b in another side wall 804c of the chamber 804.

The valve apparatus 800 includes a valve member 820 operatively attached to an actuator 830. The valve member 820 includes a valve head 822 positioned within the chamber 804, and a rod 824 extending from the valve head 822 through the chamber 804 and the body 802 of the valve apparatus. The valve head 822 includes a top surface 822a that faces the upper wall 804a, and a bottom surface 822b which faces the bottom wall 804b of the chamber 804. A channel 822c is defined within the valve head 822 to extend across the valve head 822 between a channel inlet 822d and a channel outlet 822e. The channel inlet 822d and the channel outlet 822e being formed in walls of the valve head 822 which slide along the side walls 804c of the chamber 804 as illustrated in FIGS. 8A-C.

FIG. 8A illustrates the valve apparatus 800 in a neutral position in which the valve head 822 blocks the third internal port 812b of the outlet conduit 812. Accordingly, the valve head 822 forms an obstruction between the outlet conduit 812 and both of the first inlet conduit 808 and the second inlet conduit 810. The valve member 820 is biased to move in an upward direction into the neutral position by a biasing member 840 (e.g. a spring) positioned within the chamber 804. Therefore, in a natural ("relaxed") state corresponding to the neutral position of the valve apparatus 800, a fluid is not supplied, and a vacuum pressure is not applied through the needle 14.

FIG. 8B illustrates the valve apparatus in 800 in a first actuated position after an external force has been applied to the actuator 830. The external force will be transmitted from the actuator 830 through the valve member 820 and exerted by the valve head 822 on the biasing member 840 which may compress so that the valve head 822 will move downward within the chamber 804. As illustrated in FIG. 8B, the valve head 822 is moved within the chamber 804 so that the channel inlet 822d is aligned with the second internal port 810b, and the channel outlet 822e is aligned with the third internal port 812b of the outlet conduit 812. As a result, the second inlet conduit 810 is in fluid communication with the outlet conduit 812 through the channel 822c formed within the valve head 822. A user may hold the actuator 830 in a position corresponding to a formation of a flow path between the second inlet conduit 810 and the outlet conduit 812 so that a vacuum pressure from the vacuum device 40 will be applied through the needle 14.

FIG. 8B illustrates the valve apparatus 800 in a first actuated position after an external force has been applied to the actuator 830. The external force will be transmitted from the actuator 830 through the valve member 820 and exerted by the valve head 822 on the biasing member 840 which may compress so that the valve head 822 will move downward within the chamber 804. As illustrated in FIG. 8B, the valve head 822 is moved within the chamber 804 so that the channel inlet 822d is aligned with the second internal port 810b, and the channel outlet 822e is aligned with the third internal port 812b of the outlet conduit 812. As a result, the second inlet conduit 810 is in fluid communication with the outlet conduit 812 through the channel 822c formed within the valve head 822. A user may hold the actuator 830 in a position corresponding to a formation of a flow path between the second inlet conduit 810 and the outlet conduit 812 so that a vacuum pressure from the vacuum device 40 will be applied through the needle 14.

FIG. 8C illustrates the valve apparatus 800 in a second actuated position after an external force has been applied to the actuator 830 sufficient to compress the biasing member 840 to a minimum height within the chamber 804. In the second actuated position, the valve head 822 obstructs the second internal port 810b, the channel inlet 822d is covered by one side wall 804c, and the channel outlet 822e is covered by the other side wall 804c of the chamber 804. However, the valve head 822 is moved a distance within the chamber 804 so as not to obstruct the third internal port 812b of the outlet conduit 812. In the second actuation position, a flow path is formed through the chamber 804 by the first inlet conduit 808, the upper wall 804a of the chamber 804 and the top surface 822a of the valve head 822, and the outlet conduit 812. As a result, a pressurized supply of fluid provided from the syringe 20 may flow through the first conduit 22 into to the first inlet conduit 808 and through the chamber 804. From the chamber 804, the fluid will flow under pressure through the outlet conduit 812 and the delivery conduit 82 to the needle 14.

It will be appreciated that the first conduit 22 may be connected to the second external port 810a, and the second conduit 42 may be connected to the first external port 808a. In such a configuration, fluid will be supplied from the syringe 20 through valve apparatus 800 in the first actuated position, and a vacuum pressure with be applied through the needle 14 via the valve apparatus 800 positioned in the second actuated position.

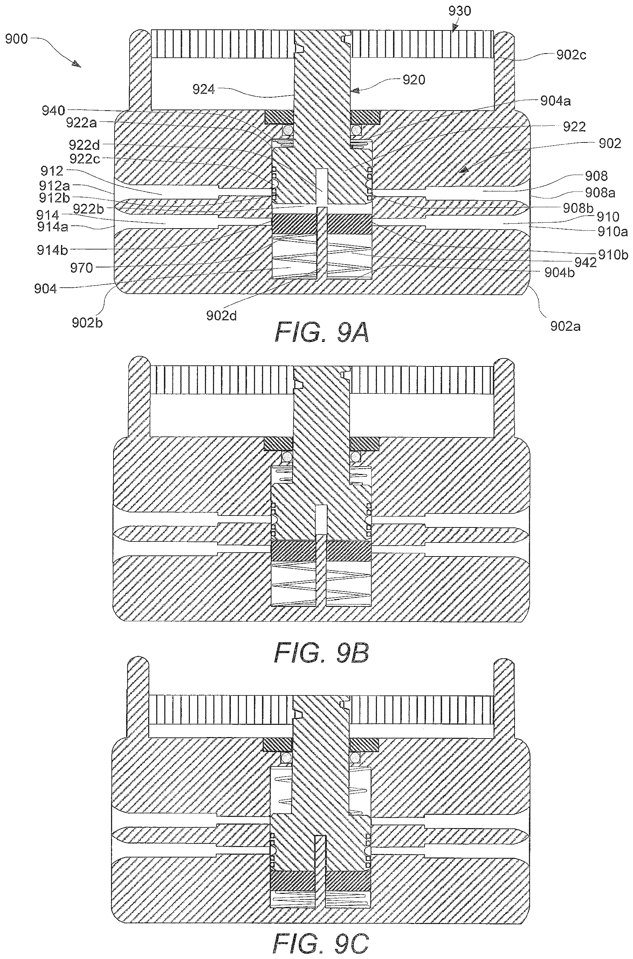

FIGS. 9A-C illustrate a front cross sectional view of a valve apparatus 900 positioned in neutral and actuated positions, according to an aspect of the present disclosure. With reference to FIG. 9A, the valve apparatus 900 includes a body 902 which defines a chamber 904, a first inlet conduit 908, a second inlet conduit 910, a first outlet conduit 912, and a second outlet conduit 914. The body includes a first wall 902a, a second wall 902b, extension walls 902c extending from the first wall 902a and the second wall 902b, and a protrusion 902d extending into the chamber 904. The chamber 904 is defined by an upper wall 904a, a bottom wall 904b, and side walls 904c formed within the body 902. The protrusion 902d may extend from the bottom wall 904b of the chamber 904.

The first inlet conduit 908 extends through the body 902 and includes a first external port 908a in the first wall 902a, and a first internal port 908b in one side wall 904c of the chamber 904. The second inlet conduit 910 extends through the body 902 and includes a second external port 910a in the first wall 902a, and a second internal port 910b in the one side wall 904c of the chamber 904. The first outlet conduit 912 extends through the body 902 from a third external port 912a in the second wall 902b of the body 902 to a third internal port 912b in another side wall 904c of the chamber 904. The second outlet conduit 914 extends through the body 902 from a fourth external port 914a in the second wall 902b to a fourth internal port 914b in the other side wall 904c of the chamber 904. Each of the third external port 912a and the fourth external portion 914a may be connected to a respective conduit branching from a hub (not shown) which is attached to a delivery conduit such as the delivery conduit 12 illustrated in FIG. 1.

The valve apparatus 900 includes a valve member 920 operatively attached to an actuator 930. The valve member 920 includes a valve head 922 positioned within the chamber 904, and a rod 924 extending from the valve head 922 through the chamber 904 and the body 902 of the valve apparatus. The valve head 922 includes a top surface 922a that faces the upper wall 904a, and a bottom surface 922b which faces the bottom wall 904b of the chamber 904. A groove 922c is formed around the valve head 922, and a recess 922d extends vertically within the valve head 922 from the bottom surface 922b.

The groove 922c is formed around the valve head 922 in a portion of the valve head 922 vertically between the top surface 922a and the bottom surface 922b. At least one sealing member 926 (e.g. an O-ring) is adjacent to each side of the groove 922c along surfaces of the valve head 922 facing the side walls 904c of the chamber 904. The sealing members 926 may prevent fluid from leaking from the groove 922c past the sealing members 926, or a loss of vacuum pressure.

The top surface 922a of the valve head 922 is attached to the upper wall 904a of the chamber 904 by a first biasing member 940. The first biasing member 940 is normally in tension and functions to draw the valve head 922 towards the upper wall 904a of the chamber 904. The first biasing member 940 pulls the valve head 922 upward via a first biasing force and thereby causes the valve member 920 to normally be in a position in which the valve head 922 obstructs the first internal port 908b and the third internal port 912b.

A second biasing member 942 is positioned in the chamber 904 extending from the bottom wall 904b. The second biasing member 942 is positioned between the bottom wall 904b and an internal stopper 970, and exerts a second biasing force on the internal stopper 970. As a result of the second biasing force, the internal stopper 970 is normally located in the chamber 904 in a position obstructing the second internal port 910b and the fourth internal port 914b. The internal stopper 970 receives the protrusion 902d in a vertical slot and the protrusion 902d guides an up and down motion of the internal stopper 970 within the chamber 904.

FIG. 9A illustrates the valve apparatus 900 in a neutral position in which the valve member 920 is pulled by the first biasing member 940 toward the upper wall 904a, and the internal stopper 970 is pushed away from the bottom wall 904b by the second biasing member 942. As a result of the action by the biasing members (940, 942) in a normal ("relaxed") state of the valve apparatus 900, the valve head 922 blocks the first internal port 908b and the third internal port 912b, and the internal stopper 970 blocks the second inlet conduit 910 and the second outlet conduit 914. Therefore, in a natural ("relaxed") state corresponding to the neutral position of the valve apparatus 900, a fluid is not supplied, and a vacuum pressure is not applied through the needle 14.

FIG. 9B illustrates the valve apparatus 900 in a first actuated position after an external force has been applied to the actuator 930. The external force is opposed by the biasing force of the first biasing member 940 which is held in tension. The external force may be transmitted from the actuator 930 through the valve member 920 and applied by the valve head 922 against the biasing force of the first biasing member 940. The first biasing member 940 may expand so that the valve head 922 moves downward within the chamber 904. As illustrated in FIG. 9B, the valve head 922 is moved within the chamber 904 so that the groove 922c is aligned with the first internal port 908b and the third internal port 912b. As a result, the second inlet conduit 910 is in fluid communication with the first outlet conduit 912 through the groove 922c formed around the valve head 922. A user may hold the actuator 930 in a position corresponding to a formation of a flow path defined by the second inlet conduit 910, the groove 922c, and the first outlet conduit 912. A pressurized supply of fluid provided from the syringe 20 may flow from the first conduit 22, through the flow path, and flow under pressure through the delivery conduit 12 to the needle 14.

An advantage of the valve apparatus 900 is that a user may know precisely when the valve head 922 is positioned so that the groove 922c is aligned with the first inlet conduit 808 and the first outlet conduit 912 due to the incorporation of the internal stopper 970. In the first actuated position, the bottom surface 922b of the valve head 922 will be in abutment with the internal stopper 970. The user will be able to feel the valve head 922 as it contacts the internal stopper 970 and know whether the valve member 920 is in the first actuated position, or has moved beyond the first actuated position. Further, additional movement of the valve head 922 will be opposed by second biasing member 942 through the internal stopper 970. The valve apparatus 900 will therefore not be undesirably sensitive to changes in external forces (as a result of a slight involuntary movement of a user's finger, for example) being applied to actuator 930. Accordingly, slight changes in the force exerted on the actuator 930 may not undesirably move valve member 920 from a position in which the flow path is formed with the first inlet conduit 908, the groove 922c, and the first outlet conduit 912.

FIG. 9C illustrates the valve apparatus 900 in a second actuated position. As an external force is applied to the actuator 930 that moves the combination of the valve head 922 and the internal stopper 970, the protrusion 902d will be received in the recess 922d. Accordingly, the protrusion 902d of the valve apparatus 900 may guide the movement of both the internal stopper 970 and the valve head 922 within the chamber 904. The protrusion 902d and the recess 922d may be configured such that an end of the protrusion 902d comes into abutment with an end of the recess 922d within the valve head 922 when the groove 922c is aligned with the second inlet conduit 910 and the second outlet conduit 914. The valve member 920 will be prevented from moving further within the chamber 904 beyond a position in which the groove 922c is aligned with the second inlet conduit 910 and the second outlet conduit 914. Therefore, a user may readily recognize the valve member 920 is in the optimal position for a flow path defined by the second inlet conduit 910, the groove 922c, and the second outlet conduit 914 to be formed. In the second actuated position, the valve head 922 covers the first internal port 908b and the third internal port 912b, and thereby prevents fluid communication between the first inlet conduit 908 and the first outlet conduit 912.

It will be appreciated that the first conduit 22 may be connected to the second external port 910a, and the second conduit 42 may be connected to the first external port 908a. In such a configuration, a vacuum pressure will be applied through the needle 14 via the valve apparatus 900 in the first actuated position, and fluid will be supplied from the syringe 20 through valve apparatus 900 positioned in the second actuated position.

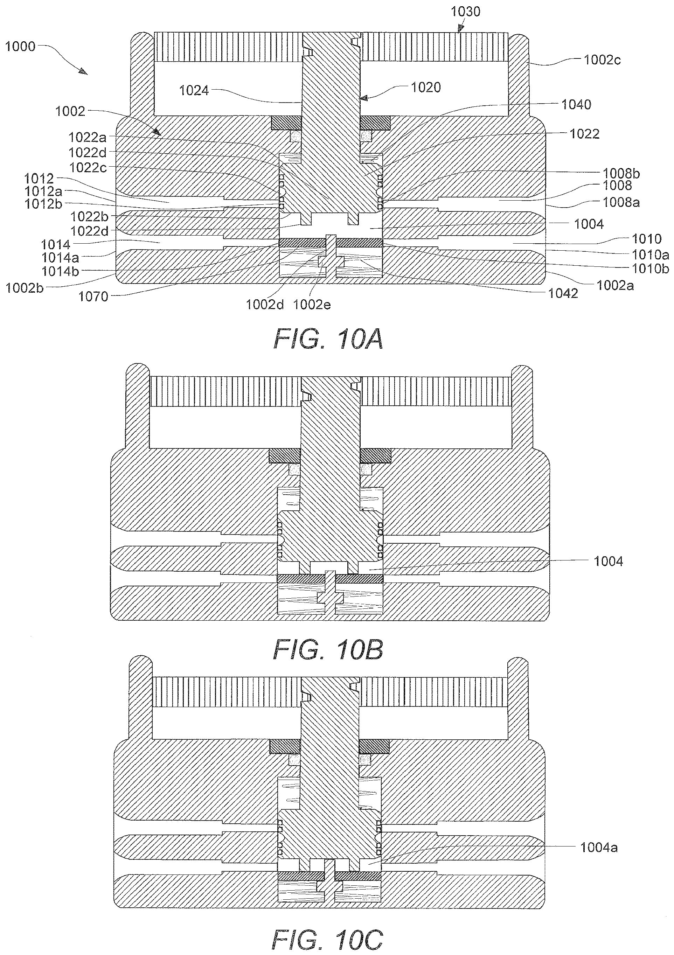

FIGS. 10A-C illustrate a front cross sectional view of a valve apparatus 1000 positioned in neutral and actuated positions, according to an aspect of the present disclosure. With reference to FIG. 10A, the valve apparatus 1000 includes a body 1002 which defines a chamber 1004, a first inlet conduit 1008, a second inlet conduit 1010, a first outlet conduit 1012, and a second outlet conduit 1014. The body includes a first wall 1002a, a second wall 1002b, extension walls 1002c extending from the first wall 1002a and the second wall 1002b, and a first protrusion 1002d extending vertically into the chamber 1004. Second protrusions 1002e may extend horizontally from opposite sides of the first protrusion 1002d. The chamber 1004 is defined by an upper wall 1004a, a bottom wall 1004b, and side walls 1004c formed within the body 1002. The first protrusion 1002d may extend from the bottom wall 1004b of the chamber 1004.

The first inlet conduit 1008 extends through the body 1002 and includes a first external port 1008a in the first wall 1002a, and a first internal port 1008b in one side wall 1004c of the chamber 1004. The second inlet conduit 1010 extends through the body 1002 and includes a second external port 1010a in the first wall 1002a, and a second internal port 1010b in the one side wall 1004c of the chamber 1004. The first outlet conduit 1012 extends through the body 1002 from a third external port 1012a in the second wall 1002b of the body 1002 to a third internal port 1012b in another side wall 1004c of the chamber 1004. The second outlet conduit 1014 extends through the body 1002 from a fourth external port 1014a in the second wall 1002b to a fourth internal port 1014b in the other side wall 1004c of the chamber 1004. Each of the third external port 1012a and the fourth external port 1014a may be connected to a respective conduit branching from a hub (not shown) which is attached to a delivery conduit such as the delivery conduit 12 illustrated in FIG. 1.

The valve apparatus 1000 includes a valve member 1020 operatively attached to an actuator 1030. The valve member 1020 includes a valve head 1022 positioned within the chamber 1004, and a rod 1024 extending from the valve head 1022 through the chamber 1004 and the body 1002 of the valve apparatus 1000. The valve head 1022 includes a top surface 1022a that faces the upper wall 1004a, and a bottom surface 1022b which faces the bottom wall 1004b of the chamber 1004. A groove 1022c is formed around the valve head 1022, and at least one third protrusion 1022d may extend from the bottom surface 1022b of the valve head 1022 toward the bottom wall 1004b of the chamber 1004. As illustrated in FIGS. 10A-C, the valve head 1022 includes two third protrusions 1022d extending from the bottom surface 1022b.

The groove 1022c is formed around the valve head 1022 in a portion of the valve head 1022 vertically between the top surface 1022a and the bottom surface 1022b. At least one sealing member 1026 (e.g. an O-ring) is adjacent to each side of the groove 1022c along surfaces of the valve head 1022 facing the side walls 1004c of the chamber 1004. The sealing members 1026 may prevent fluid from leaking from the groove 1022c past the sealing members 1026, or a loss of vacuum pressure.

The top surface 1022a of the valve head 1022 is attached to the upper wall 1004a of the chamber 1004 by a first biasing member 1040. The first biasing member 1040 is normally in tension and functions to draw the valve head 1022 towards the upper wall 1004a of the chamber 1004. The first biasing member 1040 pulls the valve head 1022 upward via a first biasing force and thereby causes the valve member 1020 to normally be in a position in which the valve head 1022 obstructs the first internal port 1008b and the third internal port 1012b.

FIG. 10A illustrates the valve apparatus 1000 in a neutral position in which the valve member 1020 is pulled by the first biasing member 1040 toward the upper wall 1004, and a first internal stopper 1070 is pushed away from the bottom wall 1004b by the second biasing member 1042. As a result of the action by the biasing members (1040, 1042) in a normal ("relaxed") state of the valve apparatus 1000, the valve head 1022 blocks the first internal port 1008b and the third internal port 1012b, and the first internal stopper 1070 blocks the second inlet conduit 1010 and the second outlet conduit 1014. Therefore, in a natural ("relaxed") state corresponding to the neutral position of the valve apparatus 1000, a fluid is not supplied, and a vacuum pressure is not applied through the needle 14.

FIG. 10B illustrates the valve apparatus 1000 in a first actuated position after an external force has been applied to the actuator 1030. The external force is opposed by the biasing force of the first biasing member 1040 which is held in tension. The external force may be transmitted from the actuator 1030 through the valve member 1020 and applied by the valve head 1022 against the biasing force of the first biasing member 1040. The first biasing member 1040 may expand so that the valve head 1022 moves downward within the chamber 1004. As illustrated in FIG. 10B, the valve head 1022 is moved with the chamber 1004 so that the groove 1022c is aligned with the first internal port 1008b and the third internal port 1012b. As a result, the second inlet conduit 1010 is in fluid communication with the first outlet conduit 1012 through the groove 1022c formed around the valve head 1022. A user may hold the actuator 1030 in a position corresponding to a formation of a flow path with the second inlet conduit 1010, the groove 1022c, and the first outlet conduit 1012. A pressurized supply of fluid provided from the syringe 20 may flow from the first conduit 22, through the flow path, and flow under pressure through the delivery conduit 12 to the needle 14.

An advantage of the valve apparatus 1000 is that the user may know precisely when the valve head 1022 is positioned so that the groove 1022c is aligned with the first inlet conduit 1008 and the first outlet conduit 1012 due to the incorporation of the first internal stopper 1070. In the first actuated position, the third protrusions 1022d of the valve head will be in abutment with the first internal stopper 1070. The user will be able to feel the third protrusions 1022d as they contact the first internal stopper 1070 and know whether the valve member 1020 is in the first actuated position, or has moved beyond the first actuated position. Further, additional movement of the valve head 1022 will be opposed by the second biasing member 1042 through the first internal stopper 1070. The valve apparatus 1000 will therefore not be undesirably sensitive to changes in external forces (as a result of a slight involuntary movement of a user's finger for example) being applied to the actuator 1030. Accordingly, slight changes in the force exerted on the actuator 1030 may not undesirably move valve member 1020 from a position in which the flow path is formed with the first inlet conduit, the groove 1022c, and the first outlet conduit 1012.

FIG. 10C illustrates the valve apparatus 1000 in a second actuated position. As an external force is applied to the actuator 1030 that moves the combination of the valve head 1022 and the first internal stopper 1070, the first internal stopper 1070 will move closer to second protrusions 1002e extending horizontally from the first protrusion 1002d. Once the first internal stopper 1070 contacts the second protrusions 1002e, neither the first internal stopper 1070 nor the valve member 1020 will be able to move further downward within the chamber 1004. Thus, the second protrusions 1002e provide a second internal stopper. In the second actuated position, the second inlet conduit 1010 is in fluid communication with the second outlet conduit 1014 through a passage defined within the chamber 1004 by the bottom surface 1022b of the valve head 1022 and a top surface of the first internal stopper 1070. Accordingly, a flow path is formed by the second inlet conduit 1010, the passage, and the second outlet conduit 1014. Further, the valve member 1020 will be prevented from moving further within the chamber 1004 by second protrusions 1002e. Therefore, a user may readily recognize the valve member 1020 is in the optimal position for the flow path including the second inlet conduit 1010 and the fourth conduit 1014 to be formed.

It will be appreciated that the first conduit 22 may be connected to the second external port 1010a, and the second conduit 42 may be connected to the first external port 1008a. In such a configuration, a vacuum pressure with be applied through the needle 14 via the valve apparatus 1000 in the first actuated position, and fluid will be supplied from the syringe 20 through valve apparatus 1000 positioned in the second actuated position.

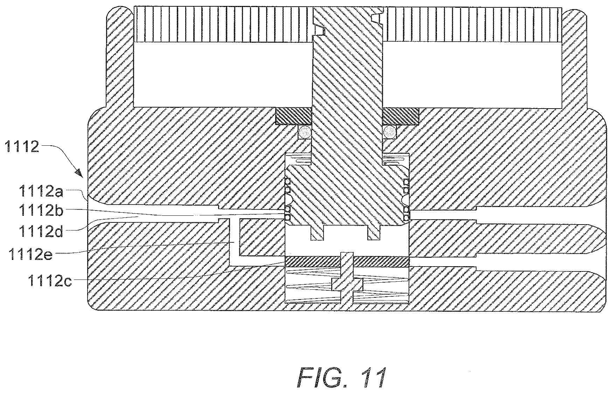

FIG. 11 illustrates a cross-sectional view of a modified version of the valve apparatus 1000 illustrated in FIGS. 10A-C. In FIG. 11, an outlet conduit 1112 is provide and includes an external port 1112a, a first internal port 1112b, and a second internal port 1112c. The external port 1112a is in fluid communication with the first internal port 1112b through a main branch 1112d of the outlet conduit 1112. In addition, the external port 1112a is in fluid communication with the second internal port 1112c through a secondary branch 1112e. Accordingly, a hub connecting to two external ports is not necessary in order for the delivery conduit 12 to be in fluid communication with multiple flow paths formed by respective internal ports. It will be appreciated that the outlet conduit 1112 may also be provided in the valve apparatus illustrated in FIGS.9A-C.

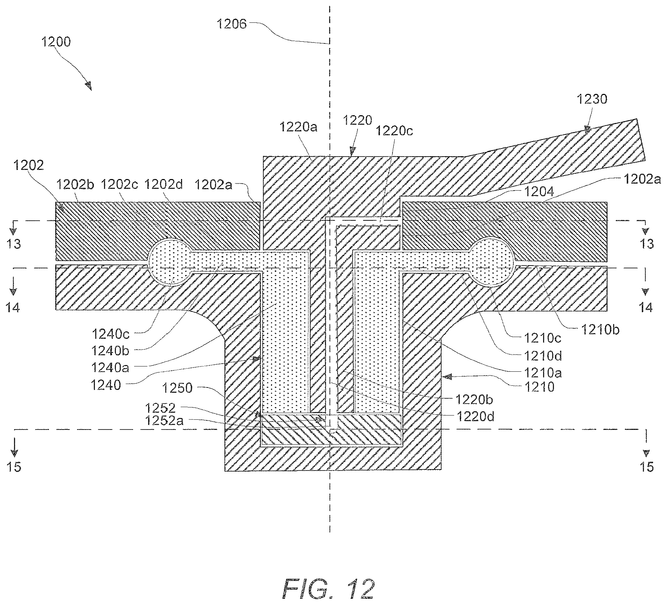

FIG. 12 illustrates a cross sectional view of a valve apparatus 1200, according to an aspect of the present disclosure. The valve apparatus 1200 includes a first body 1202 mounted on a second body 1210, and a first valve member 1220 that extends through a chamber 1204 defined by the first body 1202. An actuator 1230 extends from the first valve member 1220 above the first body 1202. The actuator 1230 may be integrally formed with the first valve member 1220. Alternatively, the actuator 1230 may include a handle or lever, for example, which is attached to the first valve member 1220 by a fastening mechanism (e.g. a screw, a snap fit connection, a slot receiving a protrusion, etc.).

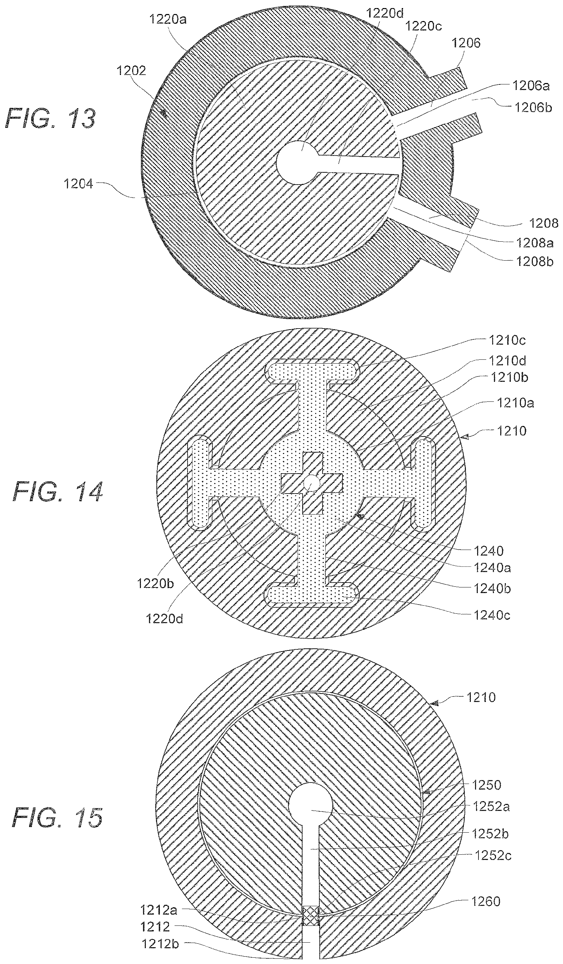

The first body 1202 includes a first vertical wall 1202a which faces the first valve member 1220, and a first horizontal wall 1202b that faces the second body 1210. At least one first receiving wall 1202c is formed between the first horizontal wall 1202b and a first recessed horizontal wall 1202d. As illustrated in FIG. 14, described in more detail below, the first body 1202 includes four first receiving walls 1202c equally spaced around an axis 1201 of the valve apparatus 1200.

The second body 1210 includes a second vertical wall 1210a which faces a portion of the first valve member 1220 positioned within the second body 1210, and a second horizontal wall 1210b that faces the first body 1202. The second horizontal wall 1210b may be attached to the first horizontal wall 1202b. At least one second receiving wall 1210c is formed between the second horizontal wall 1210b and a second recessed horizontal wall 1210d. A second receiving wall 1210c is positioned opposite to each first receiving wall 1202c in the first body 1202. Each receiving wall (1202c, 1210c) may include a flat portion extending in a radial direction relative to the axis 1201, and a recessed portion extending radially from the flat portion.

The first valve member 1220 includes a valve head 1220a positioned within the chamber 1204. The valve head 1220a has a circular cross section such that the first vertical wall 1202a provides a circumferential wall which surrounds the valve head 1220a. A valve stem 1220b extends from the valve head 1220a and into a recess within the second body 1210 defined by the second vertical wall 1210a. As illustrated in FIG. 14, a cross section of the valve stem 1220b is cross-shaped. A first channel 1220c extends through the valve head 1220a parallel to the first horizontal wall 1202b. According to one aspect of the present disclosure, a portion of the valve head 1220a defining an end of the first channel 1220c may extend from an outer surface of the valve head 1220a so as to slide along the first vertical wall 1202a in sealing abutment therewith. A second channel 1220d extends from an end of the first channel 1220c in the valve head 1220a and through the valve stem 1220b.

An elastic member 1240 is positioned within the second body 1210 surrounding the valve stem 1220b. The elastic member 1240 may be formed from an elastic material (e.g. silicone, rubber, etc.) and include a main body 1240a extending within the second body 1210 along the axis 1201 of the valve apparatus 1200. The elastic member 1240 may include arms 1240b extending from the main body 1240a parallel to the first horizontal wall 1202b and the second horizontal wall 1210b. A stopper member 1240c may extend from each arm 1240b and each stopper member 1240c may be positioned between one of the first receiving walls 1202c and a corresponding second receiving wall 1210c.

The first valve member 1220 and the elastic member 1240 may be positioned on top of a second valve member 1250. A connecting conduit defined by the second valve member 1250 may include a third channel 1252a extending vertically from a surface of the second valve member 1250 that faces the first valve member 1220 and the elastic member 1240. The third channel 1252a may be aligned with the second channel 1220d extending through the valve stem 1220b.

FIGS. 13-15 illustrate top cross sectional views of the valve apparatus 1200, taken along section lines 13-13, 14-14, and 15-15, respectively. As illustrated in FIG. 13, the first body 1202 defines a first inlet conduit 1206 and a second inlet conduit 1208. The first inlet conduit 1206 extends radially from a first internal port 1206a formed in the first vertical wall 1202a to a first external port 1206b. The second inlet conduit 1208 extends radially from a second internal port 1208a formed in the first vertical wall 1202a to a second external port 1208b. A syringe or a vacuum source, such as the syringe 20 and the vacuum device 40 illustrated in FIG. 1, may be connected to either of the first external port 1206b or the second external portion 1208b. As will be explained, the first valve member 1220 may be rotated to align the first channel 1220c with either of the first internal port 1206a and the second internal port 1208a.

As illustrated in FIG. 14, each stopper member 1240c is positioned in a respective second receiving wall 1210c of the second body 1210. The cross shaped cross section of the valve stem 1220b is completely surrounded by the elastic member 1240. According to an aspect of the present disclosure, the elastic member 1240 may be formed around the valve stem 1220b. For example, the valve stem 1220b may be positioned within a container defining a space substantially the same size as the recess defined by the second vertical wall 1210a, and an elastic material in liquid form may be supplied into to the container around the valve stem 1220b and allowed to harden. Alternatively, the elastic member 1240 may be formed independently of the first valve member 1220, and a portion of the elastic member 1240 in the shaped of the valve stem 1220b may be removed via punching or cutting, for example. In either formation, the elastic member 1240 may be attached or fitted relative to the walls of the first body 1202, second body 1210, and the second valve member 1250, so as to seal the recess defined within the second body 1210 by the second vertical wall 1210a.

The cross shape of the valve stem 1220b and a portion of the elastic member 1240 surrounding the valve stem 1220b promotes a grip between the two components. As a result, a rotational movement of first valve member 1220 is accompanied by a corresponding rotational movement of the elastic member 1240. It will be appreciated that a cross shape of the cross section of the valve stem 1220b, and corresponding shape of the elastic member 1240, is exemplary. The cross section of the valve stem 1220b may be other shapes which promote a grip between the elastic member 1240 and the first valve member 1220 and minimize slippage there between.

As illustrated in FIG. 15, the second body 1210 includes an outlet conduit 1212 which extends from a third internal port 1212a to a third external port 1212b, and the connecting conduit 1252 includes a fourth channel 1252b in fluid communication with the third channel 1252a. The fourth channel 1252b extends from a connection port 1252c formed in an external wall of the second valve member 1250, and receives a sleeve 1260. The sleeve 1260 is also received in the outlet conduit 1212 through the third internal port 1212a, and forms a fluid passage between the outlet conduit 1212 and the connecting conduit 1252.

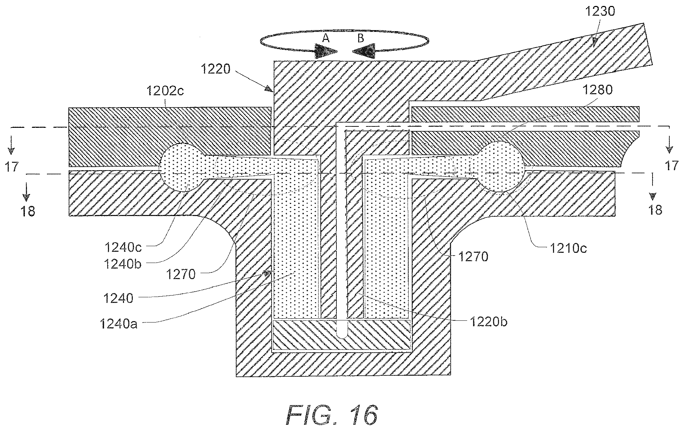

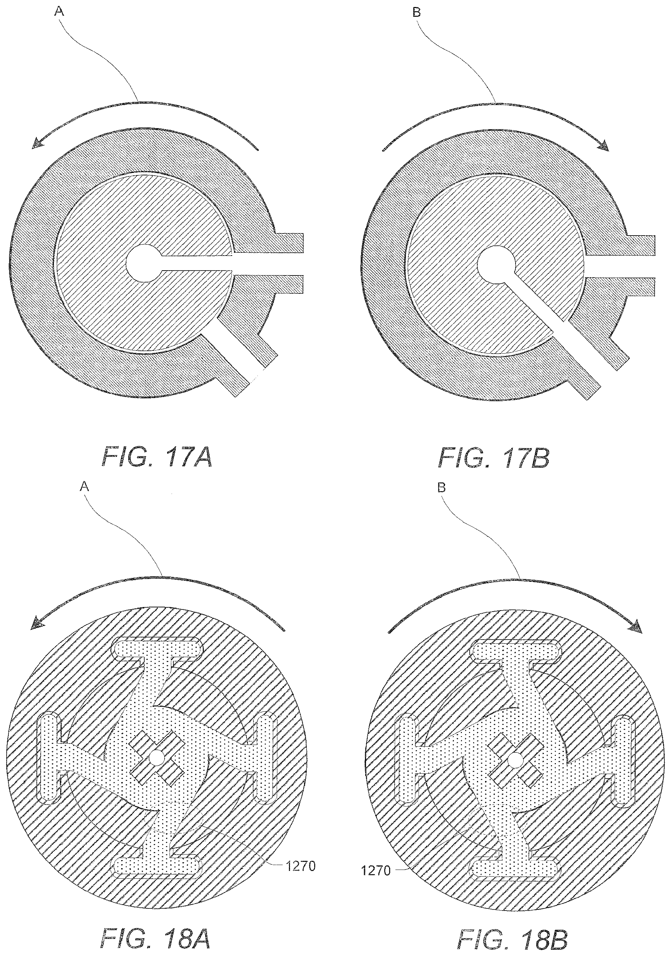

An operation of the valve apparatus 1200 of FIG 12 will be described with reference to FIGS. 16-18. FIG. 16 illustrates a cross sectional view of the valve apparatus 1200 when a force is applied to the actuator 1230. FIG. 17A-17B and FIG18A-18B, illustrate top cross sectional views of operational states of the valve apparatus of FIG. 16 take along section lines 17-17 and 18-18 respectively.

Upon rotation of the actuator 1230 in either rotational direction (A or B), the elastic member 1240 will rotate with the first valve member 1220. During rotation, the arms 1240b and the stopper members 1240c will be pressed against the first receiving wall 1220c of the first body 1202 and the second receiving wall 1210c of the second body 1210. Thus, an elastic deformation of the elastic member 1240, particularly in regions 1270 corresponding to the arms 1240b and the stopper members 1240c, will accompany the rotation of the first valve member 1220. When the actuator is rotated in direction A, as illustrated in FIGS. 17A and 18A, the first channel 1220c will be aligned and in fluid communication with the first inlet conduit 1206. When the actuator is rotated in direction B, as illustrated in FIGS. 17B and 18B, the first channel 1220c will be aligned and in fluid communication with the second inlet conduit 1208.

When a force is no longer applied to the actuator 1230, the elastic member 1240 elastically returns to an original position and applies a restoring force to the first valve member 1220 which rotates the first valve member 1220 to a neutral position illustrated in FIG. 13. As a result, when a force is not being applied to the actuator 1230, the first channel 1202c is not aligned with the first inlet conduit 1206 or the second inlet conduit 1208. Thus, the elastic member 1240 provides a biasing member that maintains the first valve member 1220 in the neutral position.

As applied to the system of FIG. 1, the delivery conduit 12 may be connected to the third external port 1212b. The syringe 20 of FIG. 1 may be connected through the first conduit 22 to the first external port 1206b, and the vacuum device 40 may be connected through the second conduit 42 to the second external port 1208b, or vice versa. During operation, when a user needs pressurized supply of fluid, the user may rotate the actuator from the neutral position in a direction towards the one of first inlet conduit 1206 and the second inlet conduit 1208 connected to the syringe 20. Conversely, if the user desires to apply a vacuum pressure through the needle 14, the user may rotate the actuator 1230 in an opposite direction so that the first channel is aligned with the other of the first inlet conduit 1206 and the second inlet conduit 1208, which is connected to the vacuum device 40.

According to one aspect of the present disclosure, the actuator 1230 may be positioned in angular alignment with the first channel 1220c. Thus, a user will know when the first valve member 1220 is positioned in a first or second actuated positioned illustrated in FIGS. 17A and 17A, when the actuator 1230 is aligned with the first internal port 1206a or the second internal port 1208a.

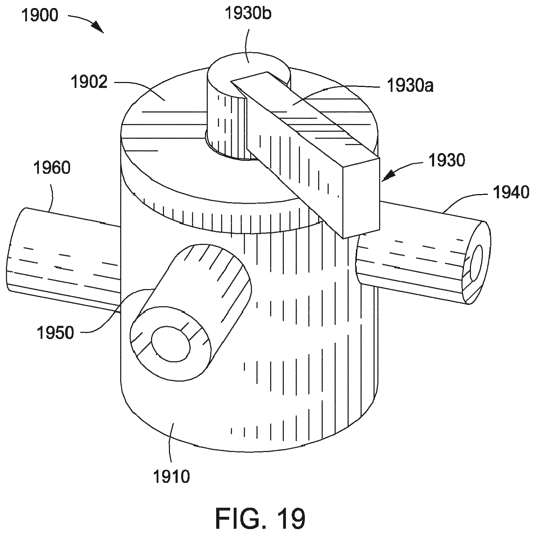

FIG. 19 illustrates a perspective view of a valve apparatus 1900, according to an aspect of the present disclosure. The valve apparatus 1900 includes a first body 1902 mounted on a second body 1910, and an actuator 1930 extending through the first body 1902. The actuator 1930 includes an actuator arm 1930a that extends from an actuator head 1930b which is mounted on top of the first body 1902. A first inlet port adapter 1940, a second inlet port adapter 1950, and an outlet port adapter 1960 extend from an outer surface of the second body 1910. A syringe or a vacuum source, such as the syringe 20 and the vacuum device 40 illustrated in FIG. 1, may be connected to either of the first inlet port adapter 1940 or the second inlet port adapter 1950.

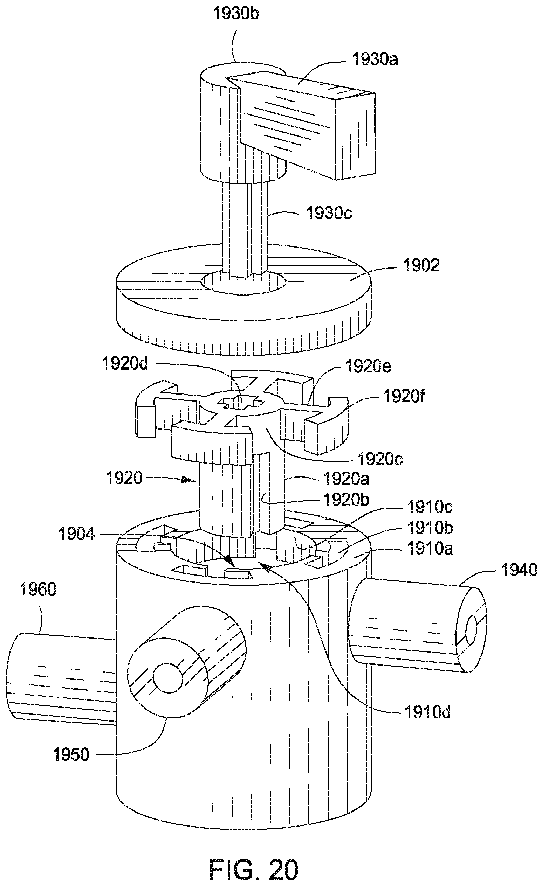

FIG. 20 illustrates an exploded view of the valve apparatus 1900 of FIG. 19. As illustrated in FIG. 20, the valve apparatus 1900 includes a valve member 1920 that is received in a chamber 1904 defined by the second body 1910 when the valve apparatus 1900 is assembled. The valve member 1920 includes a valve body 1920a that is cylindrical in shape and includes a channel 1920b formed in an outer surface thereof. It will be appreciated that the valve body 1920a may be formed in other shapes which allow the valve member 1920 to rotate in the chamber 1904. A valve head 1920c extends from an upper section of the valve body 1920a and defines a slot 1920d that extends from a surface of the valve head 1920c and through the valve body 1920a a sufficient amount to receive an extension 1930c of the actuator 1930 so that the actuator head 1930b rests on the first body 1902. The extension 1930c providing a key having a shaped that corresponds to a configuration of the slot 1920d.

The valve member 1920 further includes valve arms 1920e extending from an outer surface of the valve head 1920c. Each valve arm 1920e includes an end from which a stopper member 1920f extends. During assembly, the stopper members 1920f are received respectively, in receiving walls 1910b which are formed as recesses in a horizontal wall 1910a of the second body 1910. A first vertical wall 1910c of the second body 1910 extends from an edge of the horizontal wall 1910a and defines a valve arm chamber 1910d. The valve arm chamber 1910d extends from the chamber 1904 and provides a space which accommodates the valve arms 1920e when the valve apparatus 1900 is assembled. A diameter of the valve arm chamber 1910d may be greater than a diameter of the chamber 1904.

The extension 1930c may be cross-shaped to provide a key fitted to the slot 1920d which is also cross-shaped. With the valve member 1920 formed of elastic material, the cross shape of the extension 1930c and slot 1920d in the valve body 1920a surrounding the extension 1930c promotes a grip between the two components. As a result, a rotational movement of extension 1930c is accompanied by a corresponding rotational movement of the valve member 1920. It will be appreciated that a cross shape of the extension, and corresponding shape of the slot 1920d, is exemplary. The cross section of the extension 1930c may be other shapes which promote a grip between the actuator 1930 and the valve member 1920 and minimize slippage between the two.

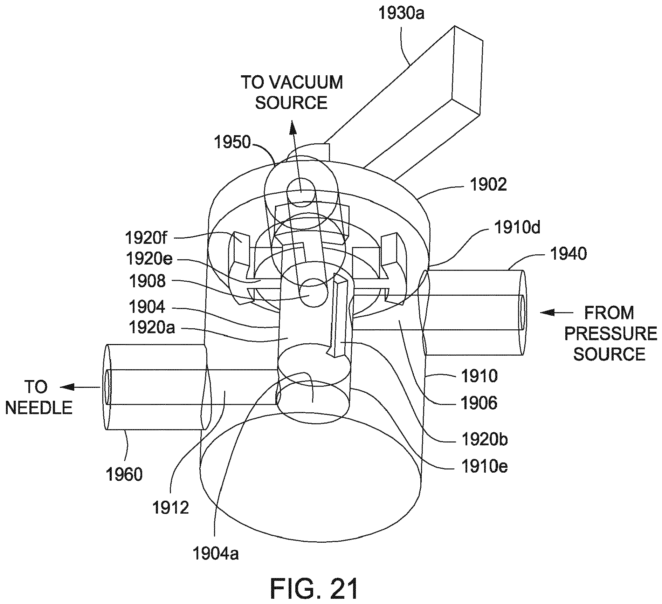

FIG. 21 illustrates a bottom perspective view of the valve apparatus of FIG. 19. As illustrated in FIG. 21, the valve body 1920a extends through a portion of the chamber 1904, and the channel 1920b is in fluid communication with a bottom end 1904a of the chamber 1904. The chamber 1904 is defined by a second vertical wall 1910e of the second body 1910. A first inlet conduit 1906 that is in fluid communication with the first inlet port adapter 1940, a second inlet conduit 1908 that is in fluid communication with the second inlet port adapter 1950, and an outlet conduit 1912 that is fluid communication with the outlet port adapter 1960, are formed within the second vertical wall 1910e of the second body 1910. The outlet conduit 1912 and the channel 1920b formed in the valve body 1920a, are in constant fluid communication with bottom end 1904a of the chamber 1904.

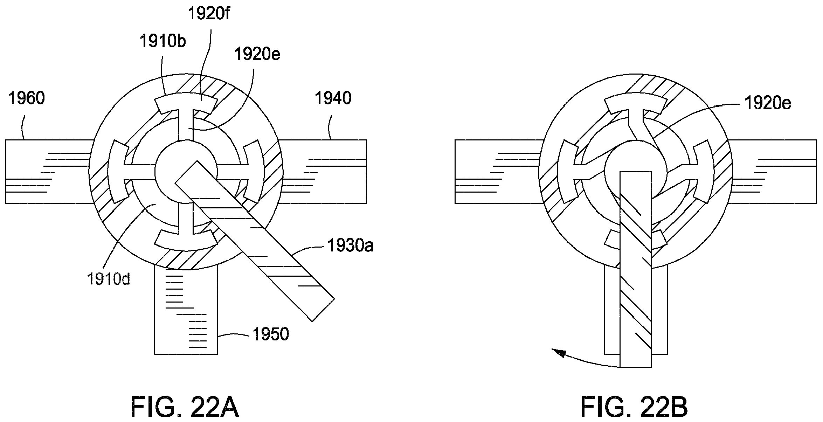

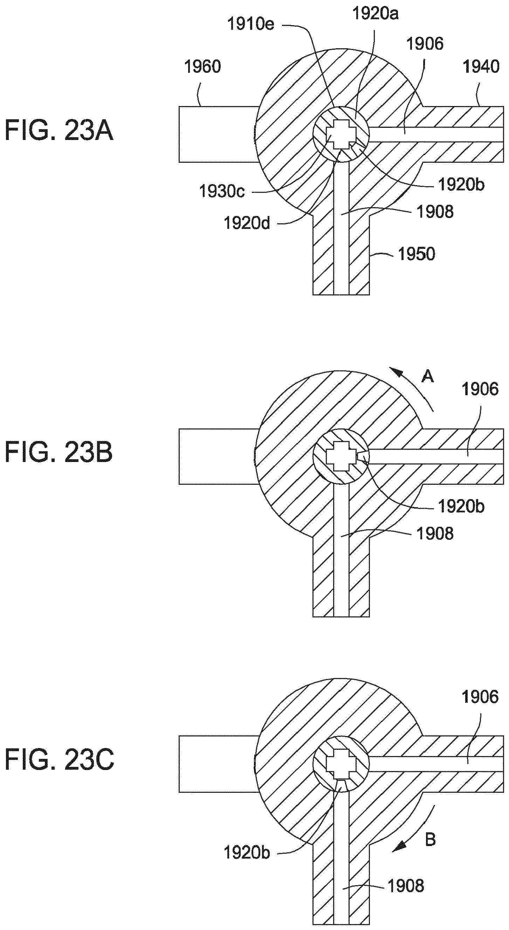

An operation of the valve apparatus 1900 of FIG 19 will be described with reference to FIGS. 22A-23C. FIGS. 22A and 22B illustrate a top view of the valve apparatus 1900 of FIG. 19. FIGS. 23A-C illustrate a cross-sectional view of the valve apparatus 1900 of FIG. 19 in neutral and actuated positions.

In a neutral position illustrated in FIG. 22A, the valve arms 1920e are straight and the stopper members 1920f are not pressed against the receiving walls 1910b. The neutral position therefore corresponds to a normal state of the valve apparatus 1900. Upon rotation of the actuator 1930, as illustrated in FIG. 22B, a rotational force will be applied to the valve body 1920a of the valve member 1920. The rotational force will be transmitted by the valve body 1920a to the valve arms 1920e which may be formed of an elastic or elastomeric material. According to one aspect of the present disclosure, the valve member may be entirely or partially formed from an elastic or elastomeric material. As a result, the valve arms 1920e are elastically deformed in the direction of the rotation of the actuator 1930 within the valve arm chamber 1910d, permitting the valve body 1920a to rotation with the rotation of the extension 1930c of the actuator 1930. During a rotation, the stopper members 1920f will be pressed against, and held inside of, the receiving walls 1910b of the second body 1910. The actuator 1930 may be rotated so that the valve apparatus 1900 may be in an actuated position in which the channel 1920b is in fluid communication with one of the first inlet conduit 1906 and the second inlet conduit 1908.

FIG. 23A illustrates the valve apparatus 1900 in the neutral position. In the neutral position, the channel 1920b faces a portion of the second vertical wall 1910e between the first inlet conduit 1906 and the second inlet conduit 1908 in a radial direction. Thus, fluid communication between the bottom end 1904a of chamber 1904 and either of the first inlet conduit 1906 and the second inlet conduit 1908 is blocked. When the actuator 1930 is rotated in direction A, as illustrated in FIG 23B, the channel 1920b will be aligned and in fluid communication with the first inlet conduit 1906. Thus, the first inlet conduit 1906 may be in fluid communication with the bottom end 1904a of the chamber 1904 and the outlet conduit 1912 with a rotation of the actuator 1930 in direction A. Where the actuator is rotated in direction B, as illustrated in FIGS. 23C, the channel 1920b will be aligned and in fluid communication with the second inlet conduit 1908. As a result of the rotation in direction B, the second inlet conduit 1908 may be in fluid communication with the bottom end 1904a of the chamber 1904 and the outlet conduit 1912.

When a force is no longer applied to the actuator 1930, the valve arms 1920e apply a restoring force to the valve body 1920b which rotates the valve member 1920 to return to the neutral position illustrated in FIG. 23A. As a result, when a force is not being applied to the actuator 1930, the valve arms 1920e combine to provide a biasing member that maintains the valve member 1920 in the neutral position.

It will be appreciated that the valve member 1920 could be provided with more than one of the channel 1920b. Further, the channel 1920b and an additional channel may be spaced along a circumference of the valve body 1920a such that a smaller amount of rotation is needed to place the first inlet conduit 1906 and the second inlet conduit 1908 in fluid communication with the bottom end 1904a of the chamber 1904.

As applied to the system of FIG. 1, the delivery conduit 12 may be connected to the outlet port adapter 1960. The syringe 20 of FIG. 1 may be connected through the first conduit 22 to the first inlet port adapter 1940, and the vacuum device 40 may be connected through the second conduit 42 to the second inlet port adapter 1950, or vice versa. During operation, when a user needs pressurized supply of fluid, the user may rotate the actuator from the neutral position in a direction towards the one of first inlet conduit 1206 and the second inlet conduit 1208 connected to the syringe 20. Conversely, if the user desires to apply a vacuum pressure through the needle 14, the user may rotate the actuator 1930 in an opposite direction so that the channel 1920b is aligned with the other of the first inlet conduit 1906 and the second inlet conduit 1908, which is connected to the vacuum device 40.

The valve apparatus (100, 600, 700, 800, 900, 1000, 1200, 1900) according to the present disclosure, may be used in various procedures in which it is desirable to be able to easily control a supply of a fluid and an application of a vacuum pressure (or a supply of a second fluid) through a needle. For example, any of the valve apparatus described herein may be used in the system illustrated in FIG. 1 to perform a peripheral nerve block procedure.

For the peripheral nerve block procedure, the syringe 20 and the vacuum device 40 may be prepared. This may involve aspirating a fluid (e.g. a medication or an anesthetic) with the syringe 20 and detaching the syringe 20 from the drug source. As the syringe 20 is filled, the plunger 26b is drawn back within the supply chamber 28, which compresses the biasing member 30. Although, the syringe biasing member 30 applies a force which could push the plunger 26b down within the supply chamber 28, this action is prevented by self-sealing valve 32.

Next, a distal end of the first conduit 22 may be connected to a first inlet conduit or a second inlet conduit of a valve apparatus (100, 600, 700, 800, 900, 1000, 1200, 1900) of the present disclosure. A distal end of the self-sealing valve 32 may be attached to a connector (e.g. a female luer) at a proximal end first conduit 22, and the self-sealing valve 32 will open and allow the syringe 20 to push fluid down toward the valve apparatus (100, 600, 700, 800, 900, 1000, 1200, 1900). A user, such as a clinician, may operate an actuator of the valve apparatus (100, 600, 700, 800, 900, 1000, 1200, 1900) to form a first flow path allowing the fluid to flow to the needle 14 and force air out of the delivery conduit 12 and the needle 14. Next, the user may release the actuator so the first flow path is no longer formed.

A distal end of the second conduit 42 may be attached to whichever of the first inlet conduit or a second inlet conduit of the valve apparatus (100, 600, 700, 800, 900, 1000, 1200, 1900) that is not attached to the first conduit 22. A proximal end of the second conduit 42 may be attached to the vacuum device 40, which may be a vacutainer vial that is attached to the syringe 20 via the clip device 50. In the case of a vacutainer, the vacutainer may be pressed into a vial, piercing a seal and providing a vacuum along the second conduit. According to a position of the actuator of the valve apparatus (100, 600, 700, 800, 900, 1000, 1200, 1900), the vacuum may not be depleted because a respective flow path has not been formed.

The valve apparatus (100, 600, 700, 800, 900, 1000, 1200, 1900) may be mounted on the hand 200 or attached to the ultrasound device 300, and the user may hold the ultrasound device 300 in one hand while inserting the needle 14 into a patient and guiding it toward a targeted nerve. The user may intermittently operate the actuator of the valve apparatus (100, 600, 700, 800, 900, 1000, 1200, 1900) (e.g. press or rotate the actuator) to aspirate and check for blood or CSF in the delivery conduit 12, an then operate the actuator to release a small bolus of fluid through the needle 14 (e.g. further press or release the actuator, or rotate the actuator in an opposite direction) which allows the user to better visualize the needle 14 under ultrasound.

Once the needle 14 is positioned near the target nerve, the user may ensure that the needle 14 is not accidentally positioned inside of a blood vessel by operating the actuator to aspirate. If the user sees blood or CSF, then the user knows to stop the procedure or reposition the needle 14 outside of the blood vessel or nerve. If the user is sure the needle 14 is not in a blood vessel, the user may operate the actuator of the valve apparatus (100, 600, 700, 800, 900, 1000, 1200, 1900) to allow the contents of the pressurized syringe 20 to inject around the target nerve.

One of ordinary skill will recognize an advantage of the valve apparatus (100, 600, 700, 800, 900, 1000, 1200, 1900) is that pre-loaded syringe may be used to supply a pressurized fluid without the need for a second user to provide the pressure. A biasing member and syringe may be sized to prevent injection pressures that could cause nerve damage if accidentally injected intraneurally. A further advantage is that a vacuum source may be used to aspirate rather than relying on a second user to pull back on a syringe plunger to provide a vacuum for aspiration. Further, since the valve apparatus (100, 600, 700, 800, 900, 1000, 1200, 1900) according to the present disclosure can be attached directly to an ultrasound device or either hand of a user, the user can easily toggle between injecting, aspirating, and obstructing the flow of fluid or application of a vacuum pressure through a needle. If a user prefers to vent a needle to atmosphere and watch for blood or CSF rather than using a vacuum source, the user can disconnect a vacuum source from the valve apparatus (100, 600, 700, 800, 900, 1000, 1200, 1900).

The valve apparatus (100, 600, 700, 800, 900, 1000, 1200, 1900) according to the present disclosure allows a single user to perform all tasks of an ultrasound aided injection procedure, for example, with very precise volumes of fluid injected or aspirated, and without delays or confusion that can be caused by verbal communication between users. Additionally, the valve apparatus (100, 600, 700, 800, 900, 1000, 1200, 1900) limits an injection pressure, and may prevent nerve damage if the injection is accidentally made intraneurally.

It will be appreciated that the present disclosure may include any one and up to all of the following examples.

Example 1: A valve apparatus for regulating a flow of fluid and an application of vacuum pressure, the valve apparatus comprising: a first body including a first wall and a second wall; a chamber defined within the first body; a valve member positioned in the chamber; a first conduit defined within the first body to extend through the first wall and configured to be in fluid communication with the chamber and one of a source of vacuum pressure and a source of fluid; a second conduit defined within the first body to extend through the first wall and configured to be in fluid communication with the chamber and an other of the source of vacuum pressure and the source of fluid; and an actuator operatively connected to an end of the valve member extending through the first body from the chamber, wherein the actuator is configured to position the valve member within the chamber to selectively form: a first flow path with the first conduit that extends through the chamber and the second wall of the first body, and a second flow path with the second conduit that extends through chamber and the second wall of the first body.

Example 2: The valve apparatus of Example 1, wherein the actuator is configured to be operated by a hand of a user holding the valve apparatus and simultaneously holding a needle.

Example 3: The valve apparatus of Examples 1 or 2, further comprising a flexible strap attached to the first wall and the second wall of the first body, wherein the flexible strap is configured to attach the valve apparatus to a hand of a user, and wherein the flexible strap is configured to attach the valve apparatus to an ultrasound device.

Example 4: The valve apparatus of Examples 1, 2, or 3, wherein the actuator is configured to position the valve member within the chamber to form an obstruction between the second wall and at least one of the first conduit and the second conduit.

Example 5: The valve apparatus of Example 4, wherein the actuator is a resilient actuator that is configured to be biased from moving the valve member from a position corresponding to a formation of one of the first flow path, the second flow path, and the obstruction.

Example 6: The valve apparatus of Example 4, further comprising a biasing member positioned within the first body of the valve apparatus.

Example 7: The valve apparatus of Example 6, wherein the biasing member is configured to apply a biasing force to the valve member, and wherein the valve member is biased by the biasing force from moving from a position corresponding to a formation of one of the first flow path, the second flow path, and the obstruction.

Example 8: The valve apparatus of Example 7, further comprising a third wall extending from the first wall above the chamber; and a fourth wall extending from the second wall above the chamber, wherein the actuator and the first end of the valve member are positioned between the third wall and the fourth wall.

Example 9: The valve apparatus of Example 8, further comprising a stopper movably positioned within a slot defined by one of the third wall and the fourth wall, wherein the valve member is biased by the biasing force from moving from a first position corresponding to a formation of the second flow path, wherein the valve member is moved against the biasing force into a second position corresponding to a formation of the obstruction and the stopper is configured to move through the slot into a locked position above the actuator, and wherein the stopper contacts a top surface of the actuator in the locked position to maintain the actuator from moving under the biasing force from the second position.

Example 10: The valve apparatus of Examples 8 or 9, wherein the biasing member is positioned within the chamber.

Example 11: The valve apparatus of Examples 8 or 9, wherein the biasing member is positioned between the third wall and the fourth wall of the first body above the chamber.

Example 12: The valve apparatus of Examples 2, 3, or 4, wherein a third conduit is defined by the second wall of the first body and configured to be in fluid communication with the chamber and the needle.

Example 13: The valve apparatus of Example 12, wherein a channel is defined within a head of the valve member positioned within the chamber.

Example 14: The valve apparatus of Example 13, wherein a top surface of the head of the valve member contacts an internal surface of the first body that defines an upper wall of the chamber in a first position of the valve member, and wherein the channel is blocked and the head of the valve member forms the obstruction in the first position.

Example 15: The valve apparatus of Example 14, wherein the valve member is positioned within the chamber a first distance from the internal surface in a second position of the valve member, and wherein the channel is in fluid communication with the second conduit and the third conduit to form the second flow path through the head of the valve member in the second position.

Example 16: The valve apparatus of Example 15, wherein the valve member is positioned within the chamber a second distance from the internal surface greater than the first distance in a third position of the valve member, and wherein the top surface of the head of the valve member and the internal surface of the first body form a passage within the chamber that is in fluid communication with the first conduit and the third conduit to form the first flow path in the third position.

Example 17: The valve apparatus of Example 12, wherein a fourth conduit is defined within the first body to extend through the second wall of the first body and configured to be in fluid communication with the chamber and the needle.

Example 18: The valve apparatus of Example 17, wherein a head of the valve member is positioned with the chamber, wherein an outer surface of the head of the valve member defines a groove, and wherein the head of the valve member forms the obstruction between the first conduit and the third conduit in a first position of the valve member.

Example 19: The valve apparatus of Example 18, further comprising a first biasing member positioned within the chamber and configured to apply a first biasing force on the head of the valve member, wherein the valve member is biased by the first biasing force from moving from the first position.

Example 20: The valve apparatus of Example 19, wherein a top surface of the head of the valve member contacts an internal surface of the first body that defines an upper wall of the chamber in the first position of the valve member.

Example 21: The valve apparatus of Example 19, wherein the head of the valve member is positioned within the chamber a first distance from a first internal surface of the first body that defines an upper wall of the chamber in a second position of the valve member, and wherein the groove is in fluid communication with first conduit and the third conduit to form the first flow path in the second position.

Example 22: The valve apparatus of Example 21, wherein the valve member is positioned within the chamber a second distance from the first internal surface greater than the first distance and the groove is in fluid communication with the second conduit and the fourth conduit to form the second flow path in a third position of the valve member.

Example 23: The valve apparatus of Examples 21 or 22, wherein the first biasing member is attached to the internal surface and a top surface of the head of the valve member, wherein the first biasing member is held in tension in the second position of the valve member.

Example 24: The valve apparatus of Example 23, further comprising a first protrusion extending from a second internal surface of the first body that defines a bottom of the chamber; a first stopper positioned within the chamber surrounding the first protrusion; and a second biasing member positioned within the chamber attached to the second internal surface and the first stopper, wherein the second biasing member applies a second biasing force to the first stopper to bias the first stopper from moving from a position corresponding to a formation of an obstruction between the second conduit and fourth conduit.

Example 25: The valve apparatus of Example 24, wherein the first stopper is in contact with the head of the valve member in the second position of the valve member.

Example 26: The valve apparatus of Example 25, wherein the head of the valve member defines a recess extending from a bottom surface of the head of the valve member in a position corresponding to the first protrusion.

Example 27: The valve apparatus of Example 26, wherein the head of the valve member is positioned within the chamber a second distance from the first internal surface greater than the first distance in a third position of the valve member, wherein the first stopper is moved a distance towards the second internal surface and the groove is in fluid communication with the second conduit and the fourth conduit to form the second flow path in the third position, and wherein the bottom surface of the head of the valve member is in abutment with the first stopper and the recess receives the first protrusion in the third position.

Example 28: The valve apparatus of Example 26, wherein at least one second protrusion extends from the bottom surface of the head of the valve member, and wherein the first stopper is in contact with the at least one second protrusion in the second position of the valve member.

Example 29: The valve apparatus of Example 28, wherein the head of the valve member is positioned within the chamber a second distance from the first internal surface greater than the first distance in a third position of the valve member, and wherein the first stopper is moved a distance towards the second internal surface and the second conduit and the fourth conduit are in fluid communication to form the second flow path through a passage defined by the bottom surface of the head of the valve member and the first stopper in the third position.

Example 30: The valve apparatus of Example 29, wherein the first protrusion contacts the bottom surface of the head of the valve member in the third position.

Example 31: The valve apparatus of Example 29, further comprising a second stopper positioned on the first protrusion below the first stopper, wherein the first stopper contacts the second stopper in the third position.

Example 32: The valve apparatus of Example 1, wherein the actuator includes a handle that extends from a wall of the valve member extending above the first conduit and the second conduit.

Example 33: The valve apparatus of Example 1, wherein the actuator is configured to be operated by a hand of a user holding the valve apparatus and simultaneously holding an ultrasound device.

Example 34: The valve apparatus of Example 1, wherein the first wall of the first body is perpendicular to the second wall.

Example 35: The valve apparatus of Example 34, wherein the actuator is configured to be operated by a hand of a user holding the valve apparatus and simultaneously holding an ultrasound or a needle.

Example 36: The valve apparatus of Example 35, further comprising a second body attached to the second wall of the first body, wherein the valve member extends through the second wall into a recess defined within the second body.

Example 37: The valve apparatus of Example 36, wherein a channel is defined within the valve member, wherein the channel is in fluid communication with a third conduit defined within the second body, and wherein the valve member is configured to rotate within the chamber of the first body and the recess of the second body.

Example 38: The valve apparatus of Example 37, further comprising a biasing member positioned with the second body and between the first body and the second body, wherein the biasing member maintains the valve member in a first position, wherein the channel is positioned between the first conduit and the second conduit along a rotational direction of movement of the valve member in the first position.

Example 39: The valve apparatus of Example 38, wherein the actuator is configured to rotate the valve member in a first direction a first distance from the first position to a second position, wherein the channel is in fluid communication with the first conduit in the second position.

Example 40: The valve apparatus of Example 39, wherein the biasing member is formed from an elastic material, wherein the biasing member is configured to apply a restoring force in a second direction to the valve member in the second position, and wherein the second direction is opposite to the first direction and the biasing member is configured to move the valve member from the first position to the second position with the restoring force.

Example 41: A fluid injection system, comprising: a fluid source; a vacuum source; a needle; an ultrasound device; and a valve apparatus including: a body including a first wall and a second wall, a chamber defined within the body, a valve member positioned in the chamber, a first conduit defined within the body to extend through first wall in fluid communication with the chamber and one of the fluid source and the vacuum source, a second conduit defined within the body to extend through the first wall in fluid communication with the chamber and an other of the fluid source and the vacuum source, and an actuator operatively connected to an end of the valve member extending through the body from the chamber, wherein the actuator is configured to position the valve member within the chamber to selectively form: a first flow path with the first conduit that extends through the chamber in fluid communication with the needle, and a second flow path with the second conduit that extends through chamber in fluid communication with the needle, and wherein the actuator is configured to be operated by a hand of a user holding the valve apparatus and simultaneously holding an ultrasound or the needle.

Example 42: A method of controlling a flow of fluid and an application of vacuum pressure through a needle with a valve apparatus including a body that defines a chamber, a valve member positioned in the chamber, and an actuator operatively attached to the valve member, the method comprising: removably mounting the valve apparatus to a first hand of a user; connecting a pressurized source of fluid to a first conduit of the valve apparatus that is defined within the body; connecting a source of vacuum pressure to a second conduit of the valve apparatus that is defined within the body; connecting the needle to a third conduit of the valve apparatus that is defined within the body; holding the needle with at least a first finger and a second finger of the first hand; operating the actuator of the valve apparatus with at least a third finger of the first hand to position the valve member within the chamber to selectively form: a first flow path that includes the first conduit, the chamber, and the third conduit for supplying the flow of fluid to the needle, and a second flow path that includes the second conduit, the chamber, and the third conduit for applying vacuum pressure through the needle.

Example 43: The method of controlling the flow of fluid and the application of vacuum pressure through the needle according to Example 42, further comprising: positioning the needle relative to an injection site of a patient; operating the actuator with the at least third finger to position the valve member within the chamber to a first position corresponding to a formation of the second flow path to apply the vacuum pressure to the needle and aspirate the injection site within the patient; and operating the actuator with the at least third finger to move the valve member within the chamber from the first position corresponding to the formation of the second flow path to a second position corresponding to a formation of the first flow path to supply the flow of fluid to the injection site.

Example 44: The method of controlling the flow of fluid and the application of vacuum pressure through the needle according to Example 42, further comprising: holding an ultrasound device in a second hand of the user; injecting the needle into a patient with the first hand; guiding the ultrasound device along a skin of the patient with the second hand; and guiding the needle within the patient to an injection site within the patient according to a view generated by the ultrasound device.

Example 45: The method of controlling the flow of fluid and the application of vacuum pressure to the needle according to Example 44, further comprising: operating the actuator with the at least third finger to position the valve member within the chamber to a first position corresponding to a formation of the second flow path to apply the vacuum pressure to the injection site; and operating the actuator with the at least third finger to move the valve member within the chamber from the first position corresponding to the formation of the second flow path to a position corresponding to a formation of the first flow path to supply the flow of fluid to the injection site.

Example 46: The method of controlling the flow of fluid and the application of vacuum pressure to the needle according to Example 44, further comprising: operating the actuator with the at least third finger to position the valve member within the chamber to a first position corresponding to a formation of the first flow path to supply the flow of fluid to the injection site; and operating the actuator with the at least third finger to position the valve member within chamber from the first position corresponding to the formation of the first flow path to a second position corresponding to a formation of the second flow path to apply the vacuum pressure to the injection site.

Example 47: The method of controlling the flow of fluid and the application of vacuum pressure to the needle according to Examples 43, 45, or 46, wherein the valve member is positioned a first distance from an internal surface of the body that defines an upper wall of the chamber in the first position, and wherein the valve member is positioned a second distance from the internal surface greater than the first distance in the second position.

Example 48: The method of controlling the flow of fluid and the application of vacuum pressure to the needle according to Examples 43, 45, 46, or 47, wherein operating the actuator with the at least third finger includes applying a downward force to the actuator and withdrawing the downward force.

Example 49: The method of controlling the flow of fluid and the application of vacuum pressure to the needle according to Examples 43, 45, 46, 47, or 48, wherein positioning the valve member in the first position includes moving the valve member into abutment with a stopper positioned within the chamber, and wherein positioning the valve member in the second position includes moving the valve member and the stopper within the chamber.

Example 50: The method of controlling the flow of fluid and the application of vacuum pressure to the needle according to Examples 42, 43, 44, 45, 46, 47, 48 or 49, further comprising operating the actuator with the at least third finger to position the valve member within the chamber to a position corresponding to a formation of an obstruction between the third conduit and both of the first conduit and the second conduit.

Example 51: The method of controlling the flow of fluid and the application of vacuum pressure to the needle according to Examples 43, 45, or 46, wherein operating the actuator with the at least third finger to position the valve member in the first position includes rotating the actuator in a first direction, and wherein operating the actuator with the at least third finger to position the valve member in the second position includes rotating the actuator in a second direction opposite to the first direction.

Example 52: A method of controlling a flow of fluid and an application of vacuum pressure to a needle with a valve apparatus including a body that defines a chamber, a valve member positioned in the chamber, and an actuator operatively attached to the valve member, the method comprising: removably mounting the valve apparatus to an ultrasound device; connecting a pressurized source of the fluid to a first conduit of the valve apparatus that is defined within the body; connecting a source of vacuum pressure to a second conduit of the valve apparatus that is defined within the body; connecting the needle to a third conduit of the valve apparatus that is defined within the body; holding a needle with a first hand of a user; holding a combination of the valve apparatus and the ultrasound device in a second hand of a user; operating the actuator of the valve apparatus with at least a first finger of the second hand to position the valve member within the chamber to selectively form: a first flow path that includes the first conduit, the chamber, and the third conduit for supplying the flow of fluid to the needle, and a second flow path that includes the second conduit, the chamber, and the third conduit for applying vacuum pressure to the needle.

Example 53: The method of controlling the flow of fluid and the application of vacuum pressure to the needle according to Example 52, further comprising: injecting the needle into a patient with the first hand; guiding the ultrasound device along a skin of the patient with the second hand; and guiding the needle within the patient to an injection site within the patient according to a view generated by the ultrasound device.

Example 54: A valve apparatus for regulating a flow of fluid and an application of vacuum pressure, the valve apparatus comprising: a first body; a second body attached to the first body and including a first wall and a second wall; a chamber defined within the second body; a valve member extending from the first wall of the second body and positioned in the chamber; a first conduit defined within the second body to extend through the second wall and configured to be in fluid communication with the chamber and one of a source of vacuum pressure and a source of fluid; a second conduit defined within the second body to extend through the second wall and configured to be in fluid communication with the chamber and an other of the source of vacuum pressure and the source of fluid; a third conduit defined within the second body to extend through the second wall and configured to be in fluid communication with a needle; and an actuator operatively connected to the valve member and extending through the first body from the chamber, wherein the actuator is configured to position the valve member within the chamber to selectively form: a first flow path that extends through the chamber via the first conduit and the third conduit, and a second flow path that extends through chamber and via the second conduit and the third conduit.

Example 55: The valve apparatus of Example 54, wherein the actuator includes a handle that extends from a head of the actuator mounted on the first body.

Example 55: The valve apparatus of Example 54, wherein the actuator is configured to be operated by a hand of a user holding the valve apparatus and simultaneously holding an ultrasound device.

Example 56: The valve apparatus of Example 54, wherein the first wall of the first body is perpendicular to the second wall.

Example 57: The valve apparatus of Example 56, wherein the valve member extends through a third wall of the second body into a recess defined within the second body.

Example 58: The valve apparatus of Example 54, wherein a channel is defined within the valve member, wherein the channel is in fluid communication with the third conduit, and wherein the valve member is configured to rotate within the chamber.

Example 59: The valve apparatus of Example 58, further comprising a biasing member positioned with the second body, wherein the biasing member maintains the valve member in a first position, wherein the channel is positioned between the first conduit and the second conduit along a rotational direction of movement of the valve member in the first position.

Example 60: The valve apparatus of Example 59, wherein the actuator is configured to rotate the valve member in a first direction a first distance from the first position to a second position, wherein the channel is in fluid communication with the first conduit in the second position.

Example 61: The valve apparatus of Example 60, wherein the biasing member is formed from an elastic material, wherein the biasing member is configured to apply a restoring force in a second direction to the valve member in the second position, and wherein the second direction is opposite to the first direction and the biasing member is configured to move the valve member from the first position to the second position with the restoring force.

It will be appreciated that the foregoing description provides examples of the disclosed systems and techniques. However, it is contemplated that other implementations of the disclosure may differ in detail from the foregoing examples. All references to the disclosure or examples thereof are intended to reference the particular example being discussed at that point and are not intended to imply any limitation as to the scope of the disclosure more generally. All language of distinction and disparagement with respect to certain features is intended to indicate a lack of preference for those features, but not to exclude such from the scope of the disclosure entirely unless otherwise indicated.

## Claims

1. A valve apparatus for regulating a flow of fluid and an application of vacuum pressure, the valve apparatus comprising:
a first body including a first wall and a second wall;
a chamber defined within the first body;
a valve member positioned in the chamber;
a first conduit defined within the first body to extend through the first wall and configured to be in fluid communication with the chamber and one of a source of vacuum pressure and a source of fluid;
a second conduit defined within the first body to extend through the first wall and configured to be in fluid communication with the chamber and an other of the source of vacuum pressure and the source of fluid; and
an actuator operatively connected to an end of the valve member extending through the first body from the chamber,
wherein the actuator is configured to position the valve member within the chamber to selectively form:
a first flow path with the first conduit that extends through the chamber and the second wall of the first body, and
a second flow path with the second conduit that extends through chamber and the second wall of the first body.

2. The valve apparatus of claim 1, further comprising a flexible strap attached to the first wall and the second wall of the first body,
wherein the flexible strap is configured to attach the valve apparatus to a hand of a user, and
wherein the flexible strap is configured to attach the valve apparatus to an ultrasound device.

3. The valve apparatus of claims 1 or 2, wherein the actuator is configured to position the valve member within the chamber to form an obstruction between the second wall and at least one of the first conduit and the second conduit.

4. The valve apparatus of claim 3, further comprising a biasing member positioned within the first body of the valve apparatus.

5. The valve apparatus of claims 2 or 3, wherein a third conduit is defined by the second wall of the first body and configured to be in fluid communication with the chamber and a needle.

6. The valve apparatus of claim 5, wherein a channel is defined within a head of the valve member positioned within the chamber.

7. The valve apparatus of claim 6, wherein a top surface of the head of the valve member contacts an internal surface of the first body that defines an upper wall of the chamber in a first position of the valve member, and
wherein the channel is blocked and the head of the valve member forms the obstruction in the first position.

8. The valve apparatus of claim 5, wherein a fourth conduit is defined within the first body to extend through the second wall of the first body and configured to be in fluid communication with the chamber and the needle.

9. The valve apparatus of claim 1, wherein the first wall of the first body is perpendicular to the second wall.

10. The valve apparatus of claim 9, wherein the actuator is configured to be operated by a hand of a user holding the valve apparatus and simultaneously holding an ultrasound or a needle.

11. The valve apparatus of claim 10, further comprising a second body attached to the second wall of the first body,
wherein the valve member extends through the second wall into a recess defined within the second body.

12. The valve apparatus of claim 11, wherein a channel is defined within the valve member,
wherein the channel is in fluid communication with a third conduit defined within the second body, and
wherein the valve member is configured to rotate within the chamber of the first body and the recess of the second body.

13. The valve apparatus of claim 12, further comprising a biasing member positioned with the second body and between the first body and the second body,
wherein the biasing member maintains the valve member in a first position,
wherein the channel is positioned between the first conduit and the second conduit along a rotational direction of movement of the valve member in the first position.

14. A fluid injection system, comprising:
a valve apparatus according to claim 1;
a fluid source;
a vacuum source;
a needle; and
an ultrasound device;
wherein the actuator is configured to be operated by a hand of a user holding the valve apparatus and simultaneously holding an ultrasound device or the needle.

15. A method of controlling a flow of fluid and an application of vacuum pressure through a needle with a valve apparatus including a body that defines a chamber, a valve member positioned in the chamber, and an actuator operatively attached to the valve member, the method comprising:
removably mounting the valve apparatus to a first hand of a user;
connecting a pressurized source of fluid to a first conduit of the valve apparatus that is defined within the body;
connecting a source of vacuum pressure to a second conduit of the valve apparatus that is defined within the body;
connecting the needle to a third conduit of the valve apparatus that is defined within the body;
holding the needle with at least a first finger and a second finger of the first hand;
operating the actuator of the valve apparatus with at least a third finger of the first hand to position the valve member within the chamber to selectively form:
a first flow path that includes the first conduit, the chamber, and the third conduit for supplying the flow of fluid to the needle, and
a second flow path that includes the second conduit, the chamber, and the third conduit for applying vacuum pressure through the needle.
